(19) Europäisches Patentamt
European Patent Office
Office européen des brevets

(11) Veröffentlichungsnummer: **0 041 627**
**B1**

(12) # EUROPÄISCHE PATENTSCHRIFT

(45) Veröffentlichungstag der Patentschrift: **08.08.84**

(21) Anmeldenummer: **81103659.9**

(22) Anmeldetag: **12.05.81**

(51) Int. Cl.³: **C 07 D 223/16**, A 61 K 31/55, C 07 D 403/06 // C07C97/10, C07D209/48

(54) **3H-2-Benzazepine, Zwischenprodukte und Verfahren zu ihrer Herstellung, sowie diese enthaltende Präparate.**

(30) Priorität: **16.05.80 US 150508**
**06.04.81 US 251579**

(43) Veröffentlichungstag der Anmeldung: **16.12.81 Patentblatt 81/50**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen:
**EP - A - 0 002 624**
**EP - A - 0 014 454**
**US - A - 4 022 800**

**Chemical Abstracts Band 80, NR. 25, 24. Juni 1974, Columbus, Ohio, USA A. PAWDA et al. "Photochemical transformations of small ring, heterocyclic compounds.LIII. Azirine photochemistry. Cyclization of 2-styryl-2H-azirines to benzazepines" Seiten 422, 423, Abstract Nr. 145924r**

(73) Patentinhaber: **F. HOFFMANN-LA ROCHE & CO. Aktiengesellschaft, CH-4002 Basel (CH)**

(72) Erfinder: **Fryer, Rodney Ian, 5 Eton Drive, North Caldwell N.J. 07006 (US)**
Erfinder: **Trybulski, Eugene J., 13 Homer Street, Parsippany N.J. 07054 (US)**
Erfinder: **Walser, Armin, 19 Crane Avenue, West Caldwell N.J. 07006 (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 041 627 B1**

BUNDESDRUCKEREI BERLIN

**Beschreibung**

Die vorliegende Erfindung betrifft 3H-2-Benzazepine der allgemeinen Formel

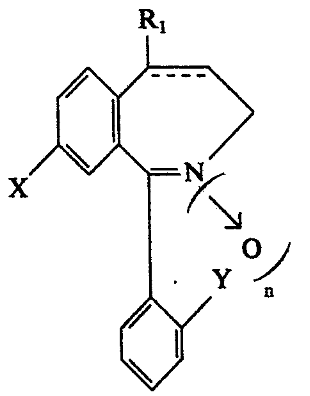

(I)

worin X Wasserstoff, Chlor oder Brom, Y Wasserstoff, Fluor oder Chlor, die gestrichelte Linie eine fakultative Bindung, n die Zahl 0 oder 1, $R_1$ Wasserstoff, Brom, Chlor, Jod oder ein Rest der Formel

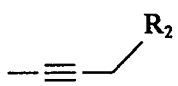

oder

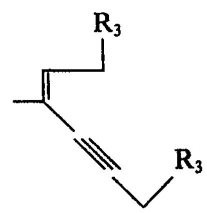

und $R_2$ Wasserstoff, $(C_1-C_7)$-Alkyl, Hydroxy, Amino, Mono-$(C_1-C_7)$-alkylamino oder Di-$(C_1-C_7)$-alkylamino und $R_3$ Hydroxy oder Amino bedeuten, mit der Maßgabe, daß X und Y nicht beide Wasserstoff bedeuten, und daß die gestrichelte Linie eine zusätzliche Bindung bedeutet, wenn $R_1$ nicht Wasserstoff bedeutet,
und pharmazeutisch annehmbare Salze davon.

In Tetrahedron Letters 1974, 33—36 werden 3H-2-Benzazepine, worin $R_1$, Y und X alle Wasserstoff bedeuten, offenbart. Diese Verbindungen erwiesen sich allerdings als inaktiv. Die strukturell ähnlichen erfindungsgemäßen Verbindungen zeigen im Gegensatz zu diesen inaktiven Verbindungen gute psychotrope Eigenschaften.

Erfindungsgemäß können die Verbindungen der Formel I und ihre pharmazeutisch annehmbaren Salze hergestellt werden, indem man

a) eine Verbindung der allgemeinen Formel

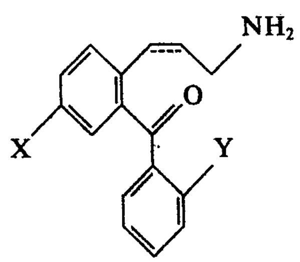

(II)

worin X und Y obige Bedeutung besitzen und die gestrichelte Linie eine fakultative Bindung bedeutet,
cyclisiert, oder

b) eine Verbindung der allgemeinen Formel

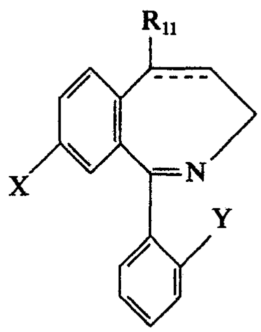

(Ia)

worin X und Y obige Bedeutung besitzen, $R_{11}$ Wasserstoff, Brom, Chlor oder Jod und die gestrichelte Linie eine fakultative Bindung bedeuten, mit der Maßgabe, daß die gestrichelte Linie eine zusätzliche Bindung bedeutet, wenn $R_{11}$ nicht Wasserstoff bedeutet,
oxydiert, oder

c) eine Verbindung der allgemeinen Formel

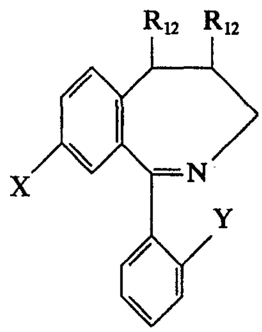

(III)

worin X und Y obige Bedeutung besitzen und $R_{12}$ Chlor, Brom oder Jod bedeutet,
dehydrohalogeniert, oder

d) eine Verbindung der allgemeinen Formel

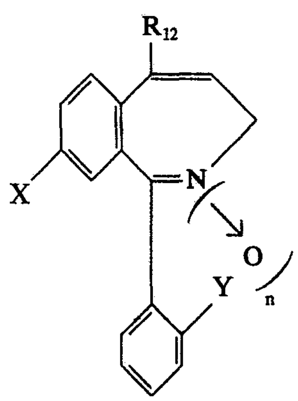

(Ib)

worin X, Y, $R_{12}$ und n obige Bedeutung besitzen,
in Gegenwart von einem Palladiumsalz, Kupfer(I)jodid, einem Organophosphin und einem sekundären oder tertiären Amin mit einer Verbindung der allgemeinen Formel

$$HC\equiv C-CH_2-R_{21} \quad (IV)$$

worin $R_{21}$ Wasserstoff, $(C_1-C_7)$-Alkyl, Hydroxy, Mono-$(C_1-C_7)$-alkylamino oder Di-$(C_1-C_7)$-alkylamino bedeutet,
umsetzt, oder

e) aus einer Verbindung der allgemeinen Formel

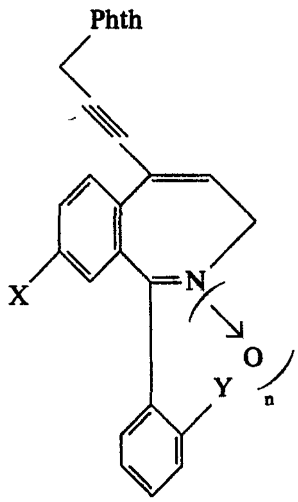

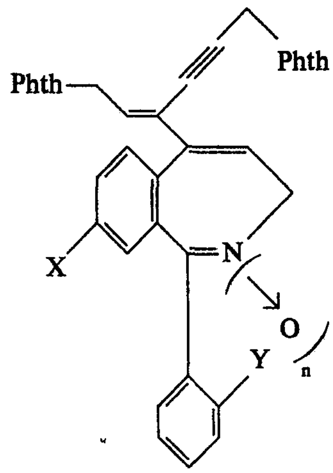

(V) oder (VI)

worin X, Y und n obige Bedeutung besitzen und Phth Phthalimido bedeutet, die Phthaloylgruppe(n) abspaltet und erwünschtenfalls

f) eine erhaltene Verbindung der allgemeinen Formel I in ein pharmazeutisch annehmbares Salz überführt.

Die obigen Verfahren zur Herstellung von Verbindungen der allgemeinen Formel I und die Herstellung der entsprechenden Ausgangsstoffe werden durch die nachfolgenden Reaktionsschemata illustriert, worin X, Y, $R_{12}$, n und Phth obige Bedeutung besitzen und $R_{22}$ Wasserstoff, Phthalimido, ($C_1-C_7$)-Alkyl, Hydroxy, Amino, Mono-($C_1-C_7$)-alkylamino oder Di-($C_1-C_7$)-alkylamino, $R_{31}$ Hydroxy, Phthalimido oder Amino und $R_4$ ($C_1-C_7$)-Alkoxy bedeuten:

Reaktionsschema I

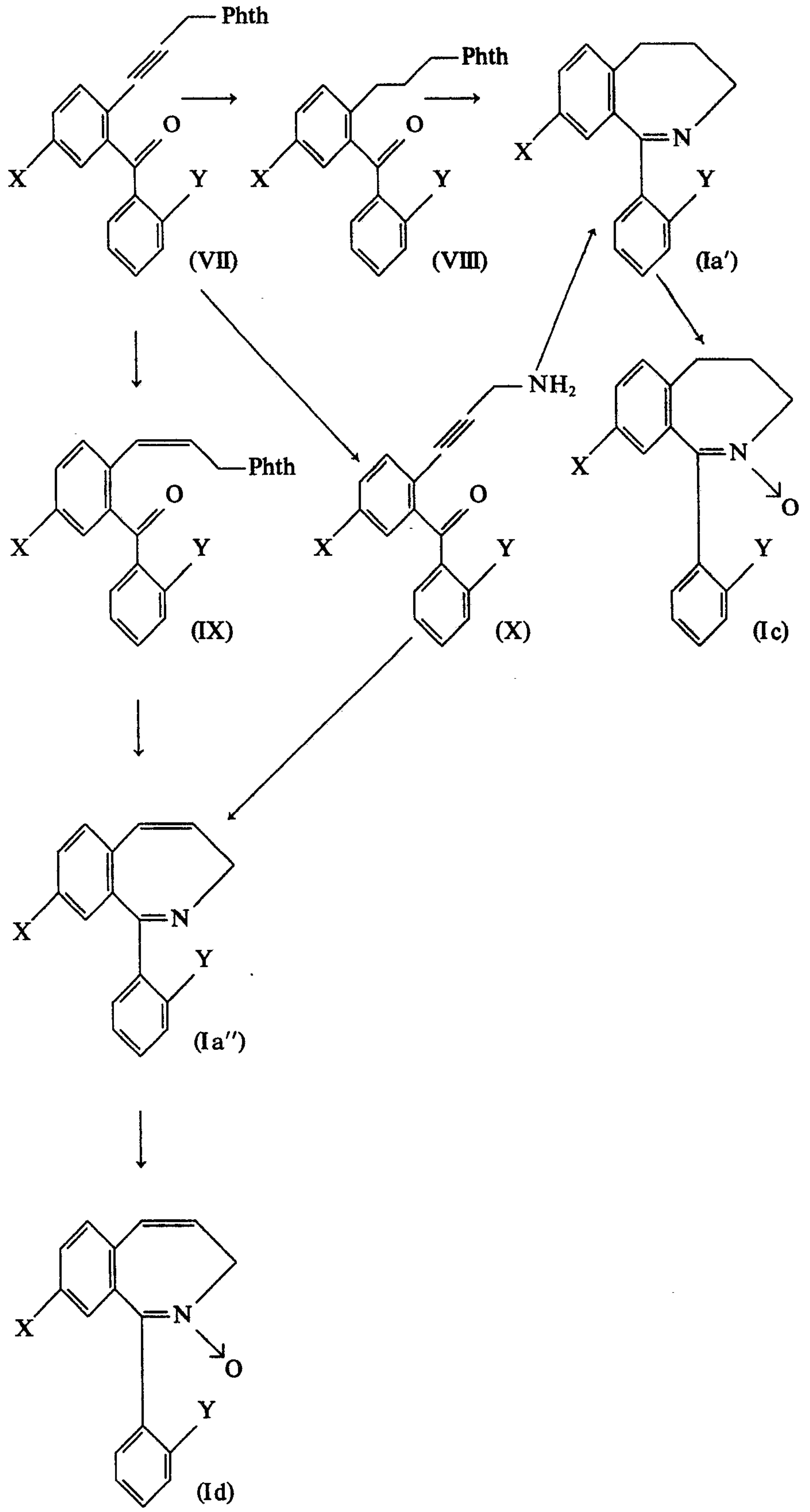

(VII) (VIII) (Ia')

(IX) (X) (Ic)

(I a'')

(I d)

Reaktionsschema II

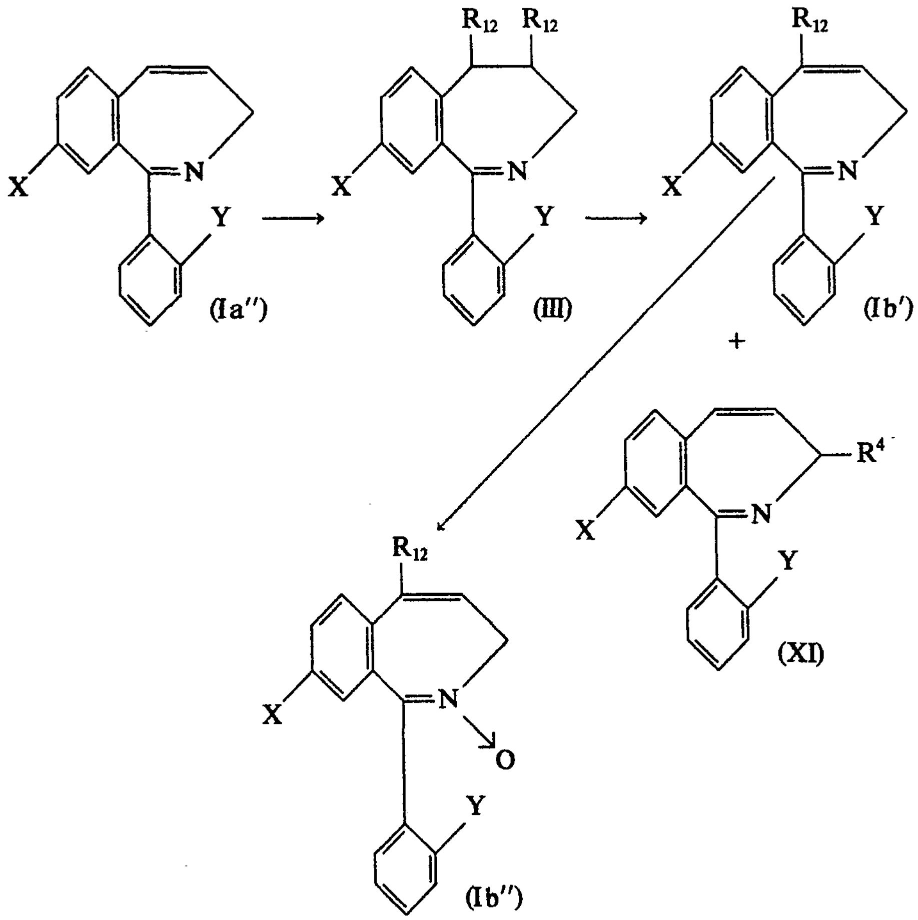

(Ia''), (III), (Ib'), (XI), (Ib'')

Reaktionsschema III

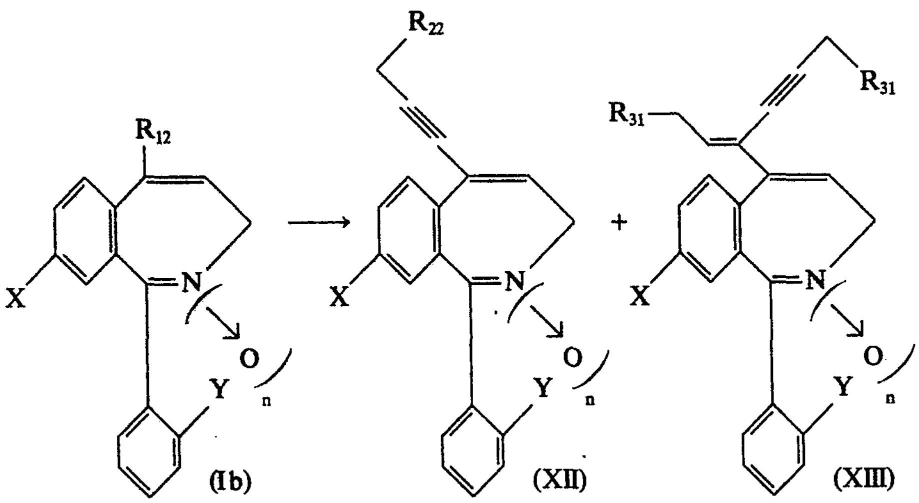

(Ib), (XII), (XIII)

Die Verbindungen der allgemeinen Formeln III, V und VI sind ebenfalls Gegenstand der vorliegenden Erfindung.

Die Verbindungen der allgemeinen Formel VII können im allgemeinen wie folgt hergestellt werden: Man diazotiert ein entsprechendes Aminobenzophenon in Gegenwart von Natriumnitrit und Schwefelsäure und isoliert das entsprechende Diazoniumsalz durch Ausfällen als Tetrafluorborat. Dieses wird in Wasser aufgeschlämmt und durch Behandeln mit wäßrigem Kaliumjodid in das entsprechende Jodbenzophenon übergeführt. Diese Reaktionen werden nach an sich bekannten Methoden durchgeführt. Anschließend wird das erhaltene Jodbenzophenon in Gegenwart einer Mischung von Palladiumchlorid, einem Organophosphin, Kupfer(I)jodid und einem sekundären Amin in einem geeigneten Lösungsmittel mit Propargylphthalimid umgesetzt, wobei man eine Verbindung der allgemeinen Formel VII erhält.

VII →VIII

Eine Verbindung der allgemeinen Formel VII wird unter Verwendung eines Übergangsmetallkatalysators, wie Raney-Nickel oder Palladiumoxyd, hydriert. Man arbeitet in einem Bereich von Atmosphärendruck bis 3,45 bar, wobei Atmosphärendruck bevorzugt wird. Geeignete Lösungsmittel sind z. B. $C_1$- bis $C_6$-Alkohole, Tetrahydrofuran, Dioxan und Toluol.

VIII →Ia' und IX →Ia''

Die Verbindungen der allgemeinen Formeln VIII und IX können mit einer wäßrigen Lösung eines niederen Alkylamins, z. B. von Methylamin, umgesetzt werden. Als Lösungsmittel kann man einen $C_1$- bis $C_4$-Alkohol verwenden, wobei Äthanol bevorzugt wird. Die Reaktion wird vorzugsweise bei Raumtemperatur durchgeführt. Das in einem ersten Schritt gebildete offenkettige Amin wird nicht isoliert; es cyclisiert spontan zu einer Verbindung der allgemeinen Formel Ia' bzw. Ia''.

Verbindungen der allgemeinen Formeln Ia' und Ia'' können auch hergestellt werden, indem man eine Verbindung der allgemeinen Formel VIII bzw. IX in einem inerten Lösungsmittel, wie Äthanol, Tetrahydrofuran, wäßriges Äthanol oder eine Mischung aus Äthanol und Chloroform, mit Hydrazin umsetzt. Die Reaktionstemperatur kann dabei in einem Bereich von Raumtemperatur bis 100° C variieren, wobei man vorzugsweise bei der Siedetemperatur des gewählten Lösungsmittels arbeitet. Das erhaltene Produkt wird mit verdünnter Mineralsäure extrahiert und anschließend durch Neutralisation des erhaltenen Salzes zurückgewonnen.

Eine dritte Methode, welche verwendet werden kann, um Verbindungen der Formeln Ia' und Ia'' herzustellen, besteht darin, daß man eine Verbindung der Formel VIII bzw. IX zuerst einer basischen Hydrolyse und anschließend einer sauren Hydrolyse unterwirft. Für die basische Hydrolyse verwendet man ein Alkalimetallhydroxyd, wie Kalium- oder Natriumhydroxyd. Für die saure Hydrolyse kann man eine 10prozentige Lösung einer Mineralsäure, wie Salzsäure, Bromwasserstoffsäure, Schwefelsäure oder Phosphorsäure, verwenden. Die Hydrolysen können in einem Temperaturbereich von Raumtemperatur bis Siedetemperatur durchgeführt werden, wobei Siedetemperatur jeweils bevorzugt wird. Organische Lösungsmittel, z. B. $C_1$- bis $C_4$-Alkohole oder Tetrahydrofuran können als Lösungsvermittler verwendet werden.

VII →IX

Eine Verbindung der allgemeinen Formel VII wird in Gegenwart eines Lindlar-Katalysators (10% Palladium auf Bariumsulfat, vorhydriert) hydriert. Man arbeitet dabei bei etwa Raumtemperatur und Atmosphärendruck. Geeignete Lösungsmittel sind z. B. $C_1$- bis $C_6$-Alkohole, Tetrahydrofuran, Dioxan oder Toluol.

VII →X

Eine Verbindung der allgemeinen Formel VII kann mit einem primären niederen Alkylamin, z. B. mit Methyl- oder Äthylamin, oder mit Hydrazin in einem mit Wasser mischbaren Lösungsmittel, wie einem $C_1$- bis $C_6$-Alkohol, einem Äther oder Dimethylformamid, umgesetzt werden. Die Reaktionstemperatur kann dabei in einem Bereich von 0° C bis 60° C variieren, wobei Raumtemperatur bevorzugt wird.

Eine Verbindung der allgemeinen Formel X kann aber auch hergestellt werden, indem man eine Verbindung der allgemeinen Formel VII zuerst einer basischen Hydrolyse und anschließend einer sauren Hydrolyse unterwirft. Für die basische Hydrolyse kann man ein Alkalimetallhydroxyd, wie Kalium- oder Natriumhydroxyd, verwenden. Für die saure Hydrolyse kann man eine 10prozentige Lösung einer Mineralsäure, wie Salzsäure, Bromwasserstoffsäure, Schwefelsäure oder Phosphorsäure, verwenden. Die Hydrolysen werden dabei in einem Temperaturbereich von Raumtemperatur bis Siedetemperatur durchgeführt, wobei Siedetemperatur bevorzugt wird. Organische Lösungsmittel, wie z. B. $C_1$- bis $C_4$-Alkohole oder Tetrahydrofuran, können als Lösungsvermittler verwendet werden.

X →Ia''

Die Herstellung einer Verbindung der allgemeinen Formel Ia'' aus einer Verbindung der allgemeinen Formel X kann in Analogie zur Herstellung einer Verbindung der allgemeinen Formel IX aus einer

Verbindung der allgemeinen Formel VII durchgeführt werden. Das in einem ersten Schritt gebildete offenkettige Amin wird nicht isoliert; es cyclisiert spontan zu einer Verbindung der allgemeinen Formel Ia″.

X→Ia′

Eine Verbindung der allgemeinen Formel X kann in Gegenwart von Raney-Nickel als Katalysator und in einem Bereich von Atmosphärendruck bis 3,45 bar, wobei Atmosphärendruck bevorzugt wird, hydriert werden. Die Reaktion wird bei etwa Raumtemperatur durchgeführt. Geeignete Lösungsmittel sind z. B. $C_1$- bis $C_6$-Alkohole, Tetrahydrofuran, Dioxan und Toluol. Das in einem ersten Schritt gebildete offenkettige Amin wird nicht isoliert; es cyclisiert spontan zu einer Verbindung der allgemeinen Formel Ia′.

Ia′→Ic, Ia″→Id und Ib′→Ib″

Die Verbindungen der allgemeinen Formeln Ia′, Ia″ und Ib′ können mit einem geeigneten Oxydationsmittel, wie m-Chlorperbenzoesäure, in einem inerten organischen Lösungsmittel, wie Methylenchlorid, umgesetzt werden. Die Reaktion kann in einem Temperaturbereich von 0° C bis Siedetemperatur des gewählten Lösungsmittels, vorzugsweise bei Raumtemperatur, durchgeführt werden.

Ia″→III

Eine Verbindung der allgemeinen Formel Ia″ kann mit einem Halogenierungsmittel, wie elementares Chlor, Brom oder Jod, in einem halogenierten Kohlenwasserstoff, wie Methylenchlorid oder Chloroform, halogeniert werden. Die Reaktion kann in einem Bereich von 0° C bis Raumtemperatur, vorzugsweise bei etwa Raumtemperatur, durchgeführt werden.

III→Ib′

Eine Verbindung der allgemeinen Formel III kann mit einem Alkalimetall-(z. B. Kalium- oder Natrium-)hydroxyd, -carbonat oder -alkoxid dehydrohalogeniert werden. Geeignete Lösungsmittel sind z. B. $C_1$- bis $C_6$-Alkohole, Tetrahydrofuran, Dioxan und Dimethylformamid. Verwendet man einen $C_1$- bis $C_6$-Alkohol als Lösungsmittel, so erhält man eine Mischung von Verbindungen der allgemeinen Formel Ib′ und XI. Die Reaktionstemperatur kann in einem Bereich von 0° C bis Siedetemperatur des gewählten Lösungsmittels variieren, wobei Raumtemperatur bevorzugt wird.

Ib→XII oder XIII

Eine Verbindung der allgemeinen Formel Ib kann mit einem monosubstituierten Acetylen der Formel $HC{\equiv}C{-}CH_2{-}R_{22}$, worin $R_{22}$ Wasserstoff, Phthalimido, $(C_1{-}C_7)$-Alkyl, Hydroxy, Mono-$(C_1{-}C_7)$-alkylamino oder Di-$(C_1{-}C_7)$-alkylamino bedeutet, umgesetzt werden. Als Beispiele für ein monosubstituiertes Acetylen seien die folgenden genannt: Propargylalkohol, Propargylphthalimid, N-Methylpropargylamin, Propin oder N,N-Dimethylpropargylamin. Die Reaktion wird in Gegenwart von Palladiumchlorid, Kupfer(I)jodid, Triphenylphosphin und einen Di- oder Trialkylamin, wie z. B. Di- oder Triäthylamin, durchgeführt. Geeignete Lösungsmittel sind z. B. halogenierte Kohlenwasserstoffe, wie Methylenchlorid oder Chloroform, und Dimethylformamid. Die Reaktionstemperatur kann dabei in einem Bereich von 0° C bis Siedetemperatur variieren, wobei Raumtemperatur bevorzugt wird.

Es wurde gefunden, daß kurze Reaktionszeiten und die Verwendung von Mono- oder Di-$(C_1{-}C_7)$-alkylamino-substituierten Acetylenen vorwiegend zu Verbindungen der allgemeinen Formel XII führt, wogegen längere Reaktionszeiten und die Verwendung von Hydroxy- und Phthalimido-substituierten Acetylenen vorwiegend zu Verbindungen der allgemeinen Formel XIII führt.

Um Verbindungen zu erhalten, worin $R_2$ oder $R_3$ Amino bedeuten, kann man in einer entsprechenden Verbindung, worin $R_{22}$ oder $R_{31}$ Phthalimido bedeutet, die Phthaloylgruppe abspalten. Diese Reaktion kann durch saure oder basische Hydrolyse oder durch Reaktion mit einem wäßrigen Monoalkylamin oder Hydrazin durchgeführt werden, und zwar in Analogie zur Herstellung von Verbindungen der allgemeinen Formel X aus Verbindungen der allgemeinen Formel VII.

Der in der vorliegenden Beschreibung verwendete Ausdruck »Alkyl« bezeichnet geradkettige oder verzweigte Kohlenwasserstoffreste mit 1—7, vorzugsweise 1—4, Kohlenstoffatomen, wie z. B. Methyl, Äthyl und Propyl.

Eine geeignete pharmazeutische Dosierungseinheit kann 1500 mg eines erfindungsgemäßen Produkts enthalten. Für die orale Verabreichung wird ein Dosierungsbereich von 1—100 mg und für die parenterale Verabreichung ein Dosierungsbereich von 1—50 mg bevorzugt. Die spezifische Dosierung richtet sich jedoch in jedem Fall nach den individuellen Bedürfnissen und dem Urteil der Person, die die erfindungsgemäßen Produkte verabreicht bzw. deren Verabreichung überwacht. Es sei an dieser Stelle betont, daß die obigen Dosierungsangaben lediglich als Beispiele dienen und in keiner Weise den Umfang der vorliegenden Erfindung oder deren Anwendbarkeit einschränken sollen.

Der in der vorliegenden Beschreibung verwendete Ausdruck »Dosierungseinheit« bezeichnet diskrete pharmazeutische Einheiten, welche zusammen mit dem benötigten pharmazeutischen Verdünnungsmittel, Träger oder Vehikel eine vorbestimmte Menge eines aktiven erfindungsgemäßen Produkts enthalten und welche als Einzeldosis in einem Säuger den gewünschten therapeutischen Effekt bewirken.

Die 3H-2-Benzazepine der allgemeinen Formel I sind nützlich als Heilmittel, insbesondere als Sedativa und Anxiolytika. Diese Verbindungen können in Form von herkömmlichen pharmazeutischen Präparaten verwendet werden. Diese Verbindungen können mit herkömmlichen organischen oder anorganischen, inerten, für die parenterale oder enterale Verabreichung geeigneten, pharmazeutischen Trägermaterialien vermischt werden. Geeignete pharmazeutische Trägermaterialien sind z. B. Wasser, Gelatine, Lactose, Stärke, Magnesiumstearat, Talk, pflanzliche Öle, Gummi arabicum, Polyalkylenglykole oder Vaseline. Die pharmazeutischen Präparate können als feste Formen, z. B. als Tabletten, Dragées, Kapseln oder Suppositorien, oder als flüssige Formen, z. B. als Lösungen, Suspensionen oder Emulsionen, verabreicht werden. Darüber hinaus können die pharmazeutischen Präparate herkömmlichen pharmazeutischen Nachbehandlungsmaßnahmen, wie Sterilisation, unterworfen werden und können herkömmliche pharmazeutische Hilfsstoffe, wie Konservierungsmittel, Stabilisierungsmittel, Netzmittel, Emulgiermittel, Salze, zur Veränderung des osmotischen Druckes, oder Puffer, enthalten. Sie können auch noch andere therapeutische wertvolle Stoffe enthalten.

Versuchsresultate, welche mit einigen erfindungsgemäßen Verbindungen im Metrazoltest erhalten wurden, illustrieren eine Eigenschaft der erfindungsgemäßen Produkte.

In periodischen Abständen wird vor dem eigentlichen Versuch ein Metrazol-Standardisierungstest durchgeführt, um diejenige Menge Metrazol zu ermitteln, welche man Mäusen verabreichen muß, um in allen Tieren konvulsive Anfälle zu erzeugen. Diese Menge beträgt gewöhnlich 125 mg/kg. Im Antimetrazoltest verabreicht man Gruppen von je 4 Mäusen verschiedene Dosen einer erfindungsgemäßen Verbindung oral. Eine Stunde später verabreicht man Metrazol (die im obigen Standardisierungstest ermittelte Menge) subcutan und beobachtet die Tiere im Hinblick auf konvulsive Anfälle. Die Tiere, welche keine konvulsiven Anfälle erleiden, werden ausgezählt. Diejenige Dosis, welche jeweils 50% der Tiere vor konvulsiven Anfällen schützt, wird als $ED_{50}$ bezeichnet. Für die Testverbindung verwendet man bei jeder Dosierung 8 Versuchstiere. Die $ED_{50}$ wird nach der Miller-Tainter-Methode ermittelt (Proc. Soc. Exp. Med. and Bio., 57, 261, 1944).

8-Chlor-5-(1-amino-2-propin-3-yl)-1-phenyl-3H-2-benzazepin-dihydrochlorid

| | |
|---|---|
| Metrazol $ED_{50}$ | 32 mg/kg p. o. |
| Toxizität | 500 mg/kg p. o. (24 h $LD_{50}$) |

8-Chlor-5-(1-amino-2-propin-3-yl)-1-(2-fluorphenyl)-3H-2-benzazepin-dihydrochlorid

| | |
|---|---|
| Metrazol $ED_{50}$ | 3 mg/kg p. o. |
| Toxizität | 500 mg/kg p. o. (24 h $LD_{50}$) |

8-Chlor-1-phenyl-3H-2-benzazepin-2-oxid

| | |
|---|---|
| Metrazol $ED_{50}$ | 5,2 mg/kg p. o. |
| Toxizität | >1000 mg/kg p. o. (24 h $LD_{50}$) |

8-Chlor-5-(1-dimethylamino-2-propin-3-yl)-1-(2-fluorphenyl)-3H-2-benzazepin-dihydrochlorid

| | |
|---|---|
| Metrazol $ED_{50}$ | 19 mg/kg p. o. |
| Toxizität | >1000 mg/kg p. o. (24 h $LD_{50}$) |

Die erfindungsgemäßen Verbindungen, worin $R_1$ Wasserstoff, Brom, Chlor oder Jod bedeutet, sind auch Zwischenprodukte in der Herstellung von anderen aktiven Benzazepinen, wie z. B. von Pyrimidobenzazepinen.

Bevorzugt werden Verbindungen der allgemeinen Formel

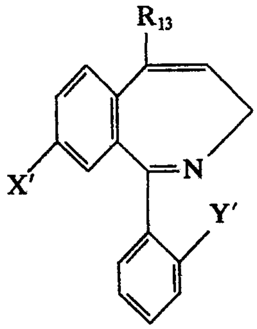

(Ie)

worin X' Chlor, Y' Wasserstoff, Fluor oder Chlor, $R_{13}$ ein Rest der Formel

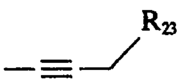

oder

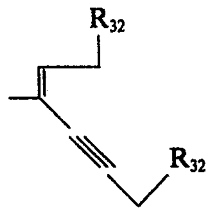

und $R_{23}$ Amino, Mono-($C_1-C_7$)-alkylamino oder Di-($C_1-C_7$)-alkylamino und $R_{32}$ Amino bedeuten.

Besonders bevorzugt sind Verbindungen der obigen Formel Ie, worin $R_{13}$ einen Rest der Formel

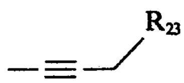

und $R_{23}$ Amino, Mono-($C_1-C_7$)-alkylamino oder Di-($C_1-C_7$)-alkylamino bedeuten.

Ganz besonders bevorzugte Verbindungen der obigen allgemeinen Formel Ie sind:

8-Chlor-5-(1-amino-2-propin-3-yl)-1-phenyl-3H-2-benzazepin und
8-Chlor-5-(1-amino-2-propin-3-yl)-1-(2-fluorphenyl)-3H-2-benzazepin.

Die obigen Verbindungen der allgemeinen Formel Ie besitzen antidepressive Eigenschaften. Diese Eigenschaften können im nachfolgend beschriebenen Imipramin Binding-Test bestimmt werden.

Man enthauptet männliche Ratten (180 g), entfernt die Gehirne und legt sie sofort auf Eis. Der Kortex wird herauspräpariert und in 8 Volumen 0,25M-Sucrose homogenisiert. Man zentrifugiert bei 2900 U/Min. (1000 x g). Der Überstand wird abdekantiert und während 60 Sekunden polytronisiert. Imipraminhydrochlorid wird mit Äthanol verdünnt (1 : 5). Jede Versuchsprobe enthält die folgenden Bestandteile: 1,8 ml Krebs-Ringer-Puffer, 20 µl Imipramin oder einen anderen Wirkstoff und 20 µl 3H-Imipramin (0,1 mMol). Man verwendet Imipramin ($10^{-3}$ M), um die nicht spezifische Bindung zu bestimmen. Man inkubiert anschließend während 15 Minuten bei Raumtemperatur. Die Proben werden während 5 Min. in ein Eisbad gestellt und anschließend filtriert. Man gibt die Filterpapiere in 13 ml Aquasol und schüttelt während 2 h. In einem Flüssigkeits-Scintillations-Spektrometer bestimmt man anschließend die auf dem Filterpapier haftende Radioaktivität.

| Verbindung | $^3$H-Imipramin-Binding $IC_{50}$ (µM) |
|---|---|
| Imipramin | 7,1 |
| 8-Chlor-5-(1-amino-2-propin-3-yl)-1-phenyl-3H-2-benzazepin-dihydrochlorid | 11,0 |
| 8-Chlor-5-(1-amino-2-propin-3-yl)-1-(2-fluorphenyl)-3H-2-benzazepin-dihydrochlorid | 20,0 |

Der Ausdruck »pharmazeutisch annehmbare Salze« bezeichnet sowohl Salze mit anorganischen als auch Salze mit organischen pharmazeutisch annehmbaren Säuren. Geeignete Säuren sind z. B. Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure und p-Toluolsulfonsäure. Unter Berücksichtigung des Standes der Technik und der Natur der Verbindung der allgemeinen Formel I können solche Salze von jedem Fachmann leicht hergestellt werden.

Die folgenden Beispiele illustrieren die vorliegende Erfindung; sie sollen sie jedoch in keiner Weise einschränken. Sofern nichts anderes angegeben ist, sind alle Temperaturen in Celsiusgraden angegeben.

### Beispiel 1

Eine Mischung aus 2 g (5 mMol) 1-[4-Chlor-2-benzoylphenyl]-3-phthalimidopropin, einem halben Teelöffel Raney-Nickel und 25 ml Tetrahydrofuran wird bei Raumtemperatur und Atmosphärendruck hydriert. Nach Aufnahme von 240 ml Wasserstoff wird vom Katalysator abfiltriert und unter vermindertem Druck zur Trockene eingedampft. Durch Kristallisieren des Rückstandes aus Äther/Petroläther erhält man das Rohprodukt als bräunlichen Festkörper vom Schmelzpunkt 94—97°. Umkristallisieren aus Äther liefert 1-[4-Chlor-2-benzoylphenyl]-3-phthalimidopropan als hellgelbe Prismen vom Schmelzpunkt 98—99°.

### Beispiel 2

Eine Mischung aus 9,6 g (23 mMol) 1-[4-Chlor-2-(2-fluorbenzoyl)phenyl]-3-phthalimidopropin, einem Teelöffel Raney-Nickel und 70 ml Tetrahydrofuran wird bei Raumtemperatur und Atmosphärendruck hydriert. Nach Aufnahme von 1,05 l Wasserstoff wird vom Katalysator abfiltriert und unter vermindertem Druck zur Trockene eingedampft. Durch Kristallisieren des Rückstandes aus Äther/Petroläther erhält man 1-[4-Chlor-2-(2-fluorbenzoyl)phenyl]-3-phthalimidopropan als bräunlichen Festkörper vom Schmelzpunkt 98—99°.

### Beispiel 3

Eine Lösung von 5,1 g (12,6 mMol) 1-[4-Chlor-2-benzoylphenyl]-3-phthalimidopropan in 100 ml Äthanol und 20 ml 3 N-Natronlauge wird während 2 Stunden unter Rückfluß zum Sieden erhitzt. Man verdünnt die erhaltene Lösung mit 60 ml 3 N-Salzsäure und erhitzt während 12 Stunden unter Rückfluß zum Sieden. Anschließend verdünnt man mit Wasser und extrahiert mit Äther. Die wäßrige Phase wird abgetrennt, mit verdünnter Natronlauge alkalisch gestellt und mit Methylenchlorid ausgeschüttelt. Die Methylenchloridphase wird über wasserfreiem Natriumsulfat getrocknet und unter vermindertem Druck zur Trockene eingedampft. Der Rückstand wird unter Nachspülen mit Methylenchlorid über 10 g Kieselgel filtriert. Das Eluat wird mit methanolischer Salzsäure sauer gestellt. Die erhaltene Lösung wird unter vermindertem Druck zur Trockene eingedampft. Durch Kristallisieren des Rückstandes aus Isopropanol/Äther erhält man einen farblosen Festkörper vom Schmelzpunkt 234—235°. Durch Umkristallisieren aus Methylenchlorid/Äther erhält man 8-Chlor-4,5-dihydro-1-phenyl-3H-2-benzazepin-hydrochlorid als farblose Nadeln vom Schmelzpunkt 234—235° (Zersetzung).

### Beispiel 4

#### Verfahren A

Eine Mischung aus 2,9 g (10 mMol) 3-Amino-1-[4-chlor-2-(2-fluorbenzoyl)phenyl]propin, $^{1}/_{4}$ Teelöffel Raney-Nickel und 50 ml Tetrahydrofuran wird bei Raumtemperatur und Atmosphärendruck hydriert. Nach Aufnahme von 430 ml Wasserstoff wird vom Katalysator abfiltriert und unter vermindertem Druck zur Trockene eingedampft. Eine Lösung des Rückstandes in überschüssiger methanolischer Salzsäure wird mit Äther verdünnt. Das ausgefallene Material wird abfiltriert und hat einen Schmelzpunkt von 176—177° (Zersetzung). Durch Umkristallisieren aus Methanol/Äther erhält man 8-Chlor-4,5-dihydro-1-(2-fluorphenyl)-3H-2-benzazepin-hydrochlorid als hellgelbe Plättchen vom Schmelzpunkt 209—215° (Zersetzung).

#### Verfahren B

Eine Lösung von 5,9 g (14 mMol) 1-[4-Chlor-2-(2-fluorbenzoyl)phenyl-3-phthalimidopropan in einer Mischung aus 100 ml Äthanol und 20 ml 3 N-Natronlauge wird während 2 Stunden unter Rückfluß zum Sieden erhitzt, mit 60 ml 3 N-Salzsäure verdünnt und während 12 Stunden unter Rückfluß zum Sieden

erhitzt. Man gießt die Reaktionsmischung auf Wasser und extrahiert mit Äther. Die saure wäßrige Phase wird abgetrennt, mit verdünnter Natronlauge alkalisch gestellt und mit Methylenchlorid ausgeschüttelt. Die Methylenchloridphase wird über wasserfreiem Natriumsulfat getrocknet und unter vermindertem Druck zur Trockene eingedampft. Durch Filtration des Rückstandes über 50 g Aluminiumoxid (Aktivitätsstufe I) unter Nachspülen mit Methylenchlorid erhält man ein Öl, das man mit überschüssiger methanolischer Salzsäure verdünnt. Nach Eindampfen unter vermindertem Druck wird der Rückstand aus Methanol/Äther kristallisiert. Man erhält 8-Chlor-4,5-dihydro-1-(2-fluorphenyl)-3H-2-benzazepinhydrochlorid das in jeder Beziehung mit authentischem Material identisch ist.

Beispiel 5

Eine Mischung aus 2,0 g (5 mMol) 1-[4-Chlor-2-benzoylphenyl]-2-phthalimidopropin, 0,1 g Palladium auf Bariumsulfat (10%, vorhydriert) und 50 mg Tetrahydrofuran wird bei Raumtemperatur und Atmosphärendruck hydriert. Nach Aufnahme von 85 ml Wasserstoff wird vom Katalysator abfiltriert und unter vermindertem Druck zur Trockene eingedampft. Kristallisieren des Rückstandes aus Äther liefert einen weißen Festkörper vom Schmelzpunkt 70—72°. Durch Umkristallisieren aus Äther erhält man 1-[4-Chlor-2-benzoylphenyl]-3-phthalimidopropen als farblose Prismen vom Schmelzpunkt 70—72°.

Beispiel 6

In Analogie zur Herstellung von 1-[4-Chlor-2-benzoylphenyl]-3-phthalimidopropen erhält man 1-[4-Chlor-2-(2-fluorbenzoyl)phenyl]-3-phthalimidopropen als farblose Nadeln vom Schmelzpunkt 117°.

Beispiel 7

Verfahren A

Eine Mischung aus 0,1 g Palladium auf Bariumsulfat (10%, vorhydriert), 27 g (0,1 Mol) 3-Amino-1-[2-benzoyl-4-chlorphenyl]propin und 100 ml Tetrahydrofuran wird bei Raumtemperatur und Atmosphärendruck hydriert.

Nach Aufnahme von 2,4 l Wasserstoff (ca. 0,1 Mol) wird vom Katalysator abfiltriert. Das Filtrat wird mit einem Überschuß an methanolischer Salzsäure und 100 ml Isopropanol verdünnt und anschließend unter vermindertem Druck zu einem kristallinen Rückstand eingedampft. Durch Verreiben des Rückstandes mit Äther/Methanol erhält man einen bräunlichen Festkörper vom Schmelzpunkt 224—226° (Zersetzung). Umkristallisieren aus Methanol/Äther liefert 8-Chlor-1-phenyl-3H-2-benzazepin-hydrochlorid als cremefarbene Prismen vom Schmelzpunkt 227—228° (Zersetzung).

Verfahren B

Eine Mischung aus 6 g (15 mMol) 1-[4-Chlor-2-benzoylphenyl]-3-phthalimidopropen, 0,9 g (18 mMol) 85prozentiges Hydrazin-hydrat und 70 ml 95prozentiges Äthanol wird während 2,5 h unter Rückfluß zum Sieden erhitzt. Man filtriert, um unlösliche Anteile zu entfernen, stellt mit eiskalter verdünnter Salzsäure sauer und extrahiert mit Äther. Die wäßrige Phase wird abgetrennt, mit verdünnter Natronlauge alkalisch gestellt und mit Methylenchlorid ausgeschüttelt. Die Methylenchloridphase wird über wasserfreiem Natriumsulfat getrocknet, mit methanolischer Salzsäure sauer gestellt, mit Isopropanol verdünnt und unter vermindertem Druck zu einem kleinen Volumen eingedampft. Durch Filtrieren erhält man 8-Chlor-1-phenyl-3H-2-benzazepin-hydrochlorid als bräunliche Prismen vom Schmelzpunkt 223—225° (Zersetzung) das in jeder Beziehung mit authentischem Material identisch ist.

Beispiel 8

In Analogie zur Herstellung von 8-Chlor-1-phenyl-3H-2-benzazepin-hydrochlorid (Verfahren A und B) erhält man 8-Chlor-1-(2-fluorphenyl)-3H-2-benzazepin-hydrochlorid als weißliche Prismen vom Schmelzpunkt 210—212° (Zersetzung).

Beispiel 9

Eine Mischung aus 4,6 g (15 mMol) 3-Amino-1-[2-(2-chlorbenzoyl)-4-chlorphenyl]propin, 0,1 g Palladium auf Bariumsulfat (vorhydriert) und 30 ml Tetrahydrofuran wird bei Raumtemperatur und Atmosphärendruck hydriert. Nach Aufnahme von 355 ml Wasserstoff wird vom Katalysator abfiltriert und unter vermindertem Druck eingedampft. Durch Kristallisieren des Rückstandes aus Äther erhält man einen cremefarbenen Festkörper vom Schmelzpunkt 113—115°. Umkristallisieren aus Äther liefert 8-Chlor-1-(2-chlorphenyl)-3H-2-benzazepin als cremefarbene Prismen vom Schmelzpunkt 117—118°.

Das entsprechende Methansulfonat kann wie folgt erhalten werden: Man versetzt eine Lösung von 8-Chlor-1-(2-chlorphenyl)-3H-2-benzazepin in Methanol mit überschüssiger 1M-Lösung von Methansulfonsäure in Methanol und versetzt anschließend mit Äther, um das Salz auszufällen. Durch Umkristallisieren aus Methanol/Äther erhält man 8-Chlor-1-(2-chlorphenyl)-3H-2-benzazepin-methansulfonat als farblose Plättchen vom Schmelzpunkt 201—202°.

Beispiel 10

In Analogie zur Herstellung von 8-Chlor-1-phenyl-3H-2-benzazepin-hydrochlorid (Verfahren A) erhält man 1-(2-Chlorphenyl)-3H-2-benzazepin-hydrochlorid als bräunliche Nadeln vom Schmelzpunkt 201—202° (Zersetzung).

Beispiel 11

Eine Lösung von 1,4 g (5,5 mMol) 8-Chlor-4,5-dihydro-1-phenyl-3H-2-benzazepin und 1,4 g (7 mMol) 85prozentige m-Chlorperbenzoesäure in 50 ml Methylenchlorid wird während 18 h bei Raumtemperatur gerührt. Die Reaktionsmischung wird mit verdünnter wäßriger Natronlauge gewaschen, über wasserfreiem Natriumsulfat getrocknet und im Vakuum zur Trockene eingedampft. Durch Kristallisieren des Rückstandes aus Äther erhält man einen farblosen Festkörper vom Schmelzpunkt 120—125°. Umkristallisieren aus Äther liefert 8-Chlor-4,5-dihydro-1-phenyl-3H-2-benzazepin-2-oxid als farblose Prismen vom Schmelzpunkt 142—143°.

Beispiel 12

Eine Mischung aus 10,7 g (42 mMol) 8-Chlor-1-phenyl-3H-2-benzazepin, 10,7 g (53 mMol) 85prozentige m-Chlorperbenzoesäure und 100 ml Methylenchlorid wird während 16 h bei Raumtemperatur gerührt. Die Reaktionsmischung wird anschließend mit kalter verdünnter, wäßriger Natronlauge und halbgesättigter Kochsalzlösung gewaschen, über wasserfreiem Natriumsulfat getrocknet und unter vermindertem Druck zur Trockene eingedampft. Durch Kristallisieren des Rückstandes aus Äther erhält man Rohprodukt vom Schmelzpunkt 130—131°. Umkristallisieren aus Äther liefert 8-Chlor-1-phenyl-3H-2-benzazepin-2-oxid als bräunliche Prismen vom Schmelzpunkt 122—123°.

Beispiel 13

In Analogie zur Herstellung von 8-Chlor-1-phenyl-3H-2-benzazepin-2-oxid erhält man 8-Chlor-1-[2-fluorphenyl]-3H-2-benzazepin-2-oxid als farblose Prismen vom Schmelzpunkt 138—139°.

Beispiel 14

In Analogie zur Herstellung von 8-Chlor-1-phenyl-3H-2-benzazepin-2-oxid erhält man 8-Chlor-1-[2-chlorphenyl]-3H-2-benzazepin-2-oxid als crèmefarbene Prismen vom Schmelzpunkt 196—197°.

Beispiel 15

In Analogie zur Herstellung von 8-Chlor-1-phenyl-3H-2-benzazepin-2-oxid erhält man 1-(2-Chlorphenyl)-3H-2-benzazepin-2-oxid als crèmefarbene Stäbchen vom Schmelzpunkt 115—116°.

Beispiel 16

Eine Lösung von 26,5 g (0,1 Mol) 8-Chlor-1-phenyl-3H-2-benzazepin in 300 ml Methylenchlorid wird tropfenweise mit 200 ml (0,18 Mol) einer 5prozentigen Lösung von Brom in Methylenchlorid versetzt. Die erhaltene Mischung wird während 1 Stunde bei Raumtemperatur gerührt, mit einem Überschuß an gesättigter Natriumcarbonatlösung verdünnt und während 15 Minuten bei Raumtemperatur gerührt. Die Methylenchloridphase wird abgetrennt, über wasserfreiem Natriumsulfat getrocknet, mit einem

Überschuß an methanolischer Salzsäure verdünnt und unter vermindertem Druck zu einem kleinen Volumen eingedampft. Man versetzt mit Äther und filtriert das ausgefallene farblose Material ab. Das rohe Salz hat einen Schmelzpunkt von 164–165°. Durch Umkristallisieren aus Methylenchlorid erhält man 8-Chlor-4,5-dibrom-4,5-dihydro-1-phenyl-3H-2-benzazepin-hydrochlorid als farblose Kristalle vom Schmelzpunkt 164–165° (Zersetzung). Es wurde gefunden, daß diese Verbindung bei 172–173° (Zersetzung) einen zweiten Schmelzpunkt besitzt.

Eine Lösung dieses Salzes in Methanol wird mit verdünnter wäßriger Natronlauge neutralisiert. Die ausgefallenen Kristalle werden abfiltriert und aus Methanol umkristallisiert. Man erhält 8-Chlor-4,5-dibrom-4,5-dihydro-1-phenyl-3H-2-benzazepin als farblose Prismen vom Schmelzpunkt 113–115°.

Beispiel 17

In Analogie zur Herstellung von 8-Chlor-4,5-dibrom-4,5-dihydro-1-phenyl-3H-2-benzazepin erhält man 8-Chlor-4,5-dibrom-4,5-dihydro-1-(2-fluorphenyl)-3H-2-benzazepin-hydrochlorid als farblosen Festkörper vom Schmelzpunkt 158–159° (Zersetzung) und die entsprechende freie Base als farblose Prismen vom Schmelzpunkt 102–103°.

Beispiel 18

In Analogie zur Herstellung von 8-Chlor-4,5-dibrom-4,5-dihydro-1-phenyl-3H-2-benzazepin erhält man 8-Chlor-4,5-dibrom-4,5-dihydro-1-(2-chlorphenyl)-3H-2-benzazepin als hellgelbe Prismen vom Schmelzpunkt 139° (Zersetzung).

Beispiel 19

Eine Lösung von 24 g (53 mMol) 8-Chlor-4,5-dibrom-4,5-dihydro-1-phenyl-3H-2-benzazepin-hydrochlorid in 1 l Methanol und 180 ml 10prozentiger wäßriger Natronlauge wird während 45 Stunden bei Raumtemperatur gerührt. Man engt die erhaltene Mischung im Vakuum auf ein kleines Volumen ein und extrahiert in Methylenchlorid. Die Methylenchloridphase wird über wasserfreiem Natriumsulfat getrocknet, mit einem Überschuß an methanolischer Salzsäure verdünnt und im Vakuum zur Trockene eingedampft. Durch Kristallisieren des Rückstandes aus Isopropanol/Äther erhält man einen weißlichen Festkörper vom Schmelzpunkt 229–230°. Durch Umkristallisieren aus Methylenchlorid erhält man 8-Chlor-5-brom-1-phenyl-3H-2-benzazepin-hydrochlorid als farblose Prismen vom Schmelzpunkt 230–235° (Zersetzung).

Die Mutterlaugen werden mit verdünnter wäßriger Natronlauge basisch gestellt und mit Methylenchlorid extrahiert. Die Methylenchloridphase wird über wasserfreiem Natriumsulfat getrocknet und im Vakuum eingedampft. Der Rückstand wird durch Säulenchromatographie an Kieselgel unter Eluieren mit Methylenchlorid und anschließend mit Äther gereinigt. Das verbleibende Öl wird in einer Lösung von Methansulfonsäure in Methanol aufgelöst. Man versetzt mit Äther und filtriert das ausgefallene Salz ab. Durch Umkristallisieren aus Methanol/Äther erhält man 8-Chlor3-methoxy-1-phenyl-3H-2-benzazepin-methansulfonat als weißliche Prismen vom Schmelzpunkt 139–140°.

Beispiel 20

Eine Mischung aus 21 g (45 mMol) 8-Chlor-4,5-dibrom-4,5-dihydro-1-(2-fluorphenyl)-3H-2-benzazepin-hydrochlorid, 40 ml Dioxan, 360 ml Methanol und 40 ml 10proz. wäßriger Natronlauge wird während 5 Stunden bei Raumtemperatur gerührt und anschließend unter vermindertem Druck auf ein kleines Volumen eingedampft. Das Konzentrat wird mit Wasser verdünnt und mit Methylenchlorid extrahiert. Die Methylenchloridphase wird über wasserfreiem Natriumsulfat getrocknet, mit Isopropanol und einem Überschuß an methanolischer Salzsäure verdünnt und unter vermindertem Druck auf ein kleines Volumen eingedampft. Der ausgefallene farblose Festkörper wird abfiltriert und hat einen Schmelzpunkt von 231–232°. Durch Umkristallisieren aus Methylenchlorid/Äther erhält man 8-Chlor-5-brom-1-(2-fluorphenyl)-3H-2-benzazepin-hydrochlorid als farblose Kristalle vom Schmelzpunkt 233–234° (Zersetzung). Das Methansulfonat des als Nebenprodukt gebildeten 8-Chlor-3-methoxy-1-(2-fluorphenyl)-3H-2-benzazepins wurde in diesem Fall nicht isoliert.

Beispiel 21

Eine Lösung von 60,0 g (0,134 Mol) 8-Chlor-4,5-dibrom-4,5-dihydro-1-(2-chlorphenyl)-3H-2-benzazepin und 75 ml 40prozentiger wäßriger Natronlauge in einer Mischung aus 300 ml Dioxan und 900 ml

Methanol wird während 4 Stunden bei Raumtemperatur gerührt. Die Mischung wird im Vakuum auf ein kleines Volumen eingeengt und anschließend mit Methylenchlorid extrahiert. Die Methylenchloridphase wird über wasserfreiem Natriumsulfat getrocknet, mit einem Überschuß an methanolischer Salzsäure und Isopropanol verdünnt und im Vakuum zur Trockene eingedampft. Der Rückstand wird aus Isopropanol/Äther kristallisiert, und der erhaltene weiße Festkörper wird zwischen Methylenchlorid und wäßrigem Natriumbicarbonat verteilt. Die Methylenchloridphase wird über wasserfreiem Natriumsulfat getrocknet und unter vermindertem Druck zu einem bernsteinfarbenen Öl eingedampft. Reinigung durch Säulenchromatographie an 250 g Kieselgel unter Eluieren mit Methylenchlorid liefert 8-Chlor-5-brom-1-(2-chlorphenyl)-3H-2-benzazepin als farblose Prismen vom Schmelzpunkt 125–127°.

Die Mutterlaugen werden zwischen Methylenchlorid und wäßrigem Ammoniak verteilt. Die Methylenchloridphase wird über wasserfreiem Natriumsulfat getrocknet und unter vermindertem Druck eingedampft. Durch Verreiben des Rückstandes mit Äther/Petroläther erhält man 8-Chlor-3-methoxy-1-(2-chlorphenyl)-3H-2-benzazepin als bräunlichen Festkörper. Durch Umkristallisieren aus Äther/Petroläther erhält man reines Material als crèmefarbene Prismen vom Schmelzpunkt 83–85°.

Beispiel 22

Eine Lösung von 6,9 g (20,7 mMol) 8-Chlor-5-brom-1-phenyl-3H-2-benzazepin und 6 g (29 mMol) 85prozentige m-Chlorperbenzoesäure in 100 ml Methylenchlorid wird während 1 Stunde bei Raumtemperatur gerührt. Die erhaltene Mischung wird mit kalter verdünnter Natronlauge gewaschen, über wasserfreiem Natriumsulfat getrocknet und unter vermindertem Druck zur Trockene eingedampft. Durch Kristallisieren des Rückstandes aus Äther erhält man einen weißen Festkörper vom Schmelzpunkt 184–185°. Umkristallisieren aus Äther liefert 8-Chlor-5-brom-1-phenyl-3H-2-benzazepin-2-oxid als farblose Prismen vom Schmelzpunkt 185–186° (Zersetzung).

Beispiel 23

Eine Mischung aus 120 mg 98prozentigem Diäthylamin, 88,6 mg (0,5 mMol) Palladiumchlorid, 262,4 mg (1 mMol) Triphenylphosphin, 95,2 mg (0,5 mMol) Kupfer(I)jodid, 8,7 g (26 mMol) 8-Chlor-5-brom-1-phenyl-3H-2-benzazepin und 25 g (0,3 Mol) 1-Dimethylamino-2-propin wird während 24 Stunden unter Argon gerührt. Man dampft unter vermindertem Druck zur Trockene ein, löst den Rückstand in Methylenchlorid und wäscht mit Wasser. Die Methylenchloridphase wird über wasserfreiem Natriumsulfat getrocknet und unter vermindertem Druck zur Trockene eingedampft. Reinigung des Rückstandes durch Säulenchromatographie an 50 g Kieselgel unter Eluieren mit Methylenchlorid und anschließend mit Äther liefert ein Öl. Das Öl wird in Methanol aufgenommen und mit einem Überschuß an äthanolischer Salzsäure verdünnt. Der ausgefallene farblose Festkörper wird abfiltriert und hat einen Schmelzpunkt von 193–195° (Zersetzung). Durch Umkristallisieren aus Methylenchlorid erhält man 8-Chlor-5-(1-dimethylamino-2-propin-3-yl)-1-phenyl-3H-2-benzazepin-dihydrochlorid (kristallisiert mit 0,25 Mol Wasser) als farblose Kristalle vom Schmelzpunkt 198–199° (Zersetzung).

Beispiel 24

Eine Mischung aus 40 ml 98prozentigem Diäthylamin, 44,3 mg (0,25 mMol) Palladiumchlorid, 131,2 mg (0,5 mMol) Triphenylphosphin, 47,6 mg (0,25 mMol) Kupfer(I)jodid, 3,5 g (9,4 mMol) 8-Chlor-5-brom-1-(2-fluorphenyl)-3H-2-benzazepin und 16 ml 1-Dimethylamino-2-propion wird während 23 Stunden bei Raumtemperatur unter Stickstoff gerührt und anschließend unter vermindertem Druck zur Trockene eingedampft. Eine Lösung des Rückstandes in Methylenchlorid wird mit Wasser gewaschen, über wasserfreiem Natriumsulfat getrocknet, mit einem Überschuß an methanolischer Salzsäure verdünnt und im Vakuum zur Trockene eingedampft. Durch Kristallisieren des Rückstandes aus Äthanol/Isopropanol erhält man einen gelben Festkörper vom Schmelzpunkt 155–156°. Umkristallisieren aus Äthanol/Äther liefert 8-Chlor-5-(1-dimethylamino-2-propin-3-yl)-1-(2-fluorphenyl)-3H-2-benzazepin-dihydrochlorid als hellgelbe Stäbchen vom Schmelzpunkt 194–195° (Zersetzung).

Beispiel 25

Eine Mischung aus 3,5 g (10 mMol) 8-Chlor-5-brom-1-phenyl-3H-2-benzazepin-2-oxid, 40 ml 98prozentigem Diäthylamin, 40 ml Dimethylformamid, 44,3 mg (0,25 mMol) Palladiumchlorid, 131,2 mg (0,5 mMol) Triphenylphosphin, 47,6 mg (0,25 mMol) Kupfer(I)jodid und 16 ml 1-Dimethylamino-2-propin wird während 24 Stunden bei Raumtemperatur unter Stickstoff gerührt und anschließend unter vermindertem Druck zur Trockene eingedampft. Eine Lösung des Rückstandes in Äther, welche Petroläther enthält, wird mit Wasser und anschließend mit kalter verdünnter Salzsäure gewaschen. Die wäßrige Lösung wird abgetrennt, mit kalter verdünnter Natronlauge alkalisch gestellt und mit Methylenchlorid ausgeschüttelt. Die Methylenchloridauszüge werden über wasserfreiem Natriumsulfat getrocknet und unter vermindertem Druck zur Trockene eingedampft. Der Rückstand kristallisiert aus Äther/Petroläther und liefert einen bräunlichen Festkörper vom Schmelzpunkt 121–123°. Durch Umkristallisieren aus Äther erhält man 8-Chlor-5-(1-dimethylamino-2-propin-3-yl)-1-phenyl-3H-2-benzazepin-2-oxid als bräunliche Prismen vom Schmelzpunkt 124–125°.

## Beispiel 26

Eine Mischung aus 40 ml 98prozentigem Diäthylamin, 44,3 mg (0,25 mMol) Palladiumchlorid, 133,2 mg (0,5 mMol) Triphenylphosphin, 47,6 mg (0,25 mMol) Kupfer(I)jodid, 3,5 g (10,5 mMol) 8-Chlor-5-brom-1-phenyl-3H-2-benzazepin und 5 ml Propargylalkohol wird während 7 Stunden unter Stickstoff bei Raumtemperatur gerührt und anschließend unter vermindertem Druck zur Trockene eingedampft. Eine Lösung des Rückstandes in Methylenchlorid wird mit Wasser gewaschen, über wasserfreiem Natriumsulfat getrocknet und unter vermindertem Druck zur Trockene eingedampft. Reinigung des erhaltenen Rückstandes durch Säulenchromatographie an 20 g Kieselgel unter Eluieren mit 20% Äther in Methylenchlorid liefert ein farbloses Öl. Dieses Öl wird in einem Überschuß an methanolischer Salzsäure aufgenommen. Man dampft unter vermindertem Druck zur Trockene ein und kristallisiert den Rückstand aus Methanol/Äther. Man erhält 8-Chlor-5-(1-hydroxy-2-propin-3-yl)-1-phenyl-3H-2-benzazepin-hydrochlorid als bräunlichen Festkörper vom Schmelzpunkt 228–229° (Zersetzung).

## Beispiel 27

Eine Mischung aus 40 ml 98prozentigem Diäthylamin, 44,3 mg (0,25 mMol) Palladiumchlorid, 131 mg (0,5 mMol) Triphenylphosphin, 47,6 mg (0,25 mMol) Kupfer(I)jodid, 3,5 g (10,5 mMol) 8-Chlor-5-brom-1-phenyl-3H-2-benzazepin und 5 ml Propargylalkohol wird während 24 Stunden bei Raumtemperatur unter Stickstoff gerührt und anschließend unter vermindertem Druck zur Trockene eingedampft. Eine Lösung des Rückstandes in Methylenchlorid wird mit Wasser gewaschen, über wasserfreiem Natriumsulfat getrocknet und unter vermindertem Druck zur Trockene eingedampft. Reinigung durch Säulenchromatographie an 20 g Kieselgel unter Eluieren mit 20% Äther in Methylenchlorid liefert ein Öl, das aus Tetrahydrofuran/Äther kristallisiert. Man erhält 8-Chlor-5-(1,6-dihydroxy-2-hexen-4-yn-3-yl)-1-phenyl-3H-2-benzazepin als bräunliche Prismen vom Schmelzpunkt 210–211° (Zersetzung).

Das entsprechende Hydrochlorid kann wie folgt hergestellt werden: Man löst den erhaltenen Stoff in einem Überschuß an methanolischer Salzsäure, versetzt mit Äther und filtriert das ausgefallene Material ab. Durch Umkristallisieren aus Methanol/Äther erhält man 8-Chlor-5-(1,6-dihydroxy-2-hexen-4-yn-3-yl)-1-phenyl-3H-2-benzazepin-hydrochlorid als crèmefarbene Plättchen vom Schmelzpunkt 290°.

## Beispiel 28

Eine gerührte Mischung aus 180 ml 98prozentigem Diäthylamin (mit Stickstoff vorbehandelt), 7,2 g (21,6 mMol) 8-Chlor-5-brom-1-phenyl-3H-2-benzazepin, 0,4 g (0,6 mMol) Dichlor-bis(triphenylphosphin)palladium(II), 0,1 g (0,5 mMol) Kupfer(I)jodid wird mit Propin gesättigt, unter einer Propinatmosphäre während 23 Stunden gerührt und anschließend unter vermindertem Druck zur Trockene eingedampft. Eine Lösung des Rückstandes in Methylenchlorid wird nacheinander mit verdünnter Natriumcarbonatlösung und Wasser gewaschen, über wasserfreiem Natriumsulfat getrocknet und unter vermindertem Druck zur Trockene eingedampft. Eine Lösung des erhaltenen Öls in Methylenchlorid wird mit einem Überschuß an äthanolischer Salzsäure verdünnt und unter vermindertem Druck eingeengt. Das erhaltene Material kristallisiert aus Äthanol/Isopropanol und liefert einen gelben Festkörper vom Schmelzpunkt 206–207° (Zersetzung). Durch Umkristallisieren aus Äthanol/Äther erhält man 8-Chlor-5-(1-propynyl)-1-phenyl-3H-2-benzazepin-hydrochlorid als hellgelbe Prismen vom Schmelzpunkt 210–211° (Zersetzung).

## Beispiel 29

Eine Mischung aus 600 ml 98prozentigem Diäthylamin, 600 ml Methylenchlorid, 24 g (65 mMol) 8-Chlor-5-brom-1-phenyl-3H-2-benzazepin-hydrochlorid, 7,2 g (10 mMol) Dichlor-bis(triphenylphosphin)palladium(II), 1,8 g (9,5 mMol) Kupfer(I)jodid und 20 g (0,11 Mol) Propargylphthalimid wird während 5,5 Stunden bei Raumtemperatur unter Stickstoff gerührt und anschließend unter vermindertem Druck zur Trockene eingedampft. Eine Lösung des Rückstandes in Methylenchlorid wird mit Wasser

gewaschen, über wasserfreiem Natriumsulfat getrocknet und unter vermindertem Druck zur Trockene eingedampft. Reinigung durch Säulenchromatographie an 450 g Kieselgel unter Eluieren mit 10% Äther in Methylenchlorid (Gradient) liefert als ersten Stoff 8-Chlor-5-(1-phthalimido-2-propin-3-yl)-1-phenyl-3H-2-benzazepin als bräunlichen Festkörper vom Schmelzpunkt 177—178°. Durch Umkristallisieren aus Methylenchlorid/Äther erhält man bräunliche Prismen vom Schmelzpunkt 185—186°.

Als zweiten Stoff wird 8-Chlor-5-(1,6-diphthalimido-2-hexen-4-yn-3-yl)-1-phenyl-3H-2-benzazepin als weißlicher Festkörper vom Schmelzpunkt 185—189° eluiert. Durch Umkristallisieren aus Acetonitril erhält man graue Nadeln vom Schmelzpunkt 196—198°.

Beispiel 30

Eine Mischung aus 40 ml 98prozentigem Diäthylamin, 50 ml Methylenchlorid, 0,6 g (0,9 mMol) Dichlor-bis(triphenylphosphin)palladium(II), 0,15 g (0,8 mMol) Kupfer(I)jodid, 3,3 g (10,8 mMol) 8-Chlor-5-brom-1-(2-fluorphenyl)-3H-2-benzazepin und 3 g (16 mMol) Propargylphthalimid wird während 24 Stunden unter Stickstoff bei Raumtemperatur gerührt und anschließend unter vermindertem Druck zur Trockene eingedampft. Eine Lösung des Rückstandes in Methylenchlorid wird mit Wasser gewaschen, über wasserfreiem Natriumsulfat getrocknet und unter vermindertem Druck zur Trockene eingedampft. Reinigung des Rückstandes durch Säulenchromatographie an 50 g Kieselgel unter Eluieren mit 5% Äther in Methylenchlorid (Gradient) liefert als Hauptfraktion einen farblosen Festkörper. Durch Umkristallisieren aus Äther erhält man 8-Chlor-1-(2-fluorphenyl)-5-(1-phthalimido-2-propin-3-yl)-3H-2-benzazepin als farblose Nadeln vom Schmelzpunkt 164—165°.

Beispiel 31

Eine Mischung aus 3,7 g (10 mMol) 8-Chlor-5-brom-1-phenyl-3H-2-benzazepin-hydrochlorid, 0,4 g (0,6 mMol) Dichlor-bis(triphenylphosphin)palladium(II), 0,2 g (1 mMol) Kupfer(I)jodid und 2 ml (Überschuß) 97prozentigem N-Methylpropargylamin in 50 ml 98prozentigem Diäthylamin und 100 ml Methylenchlorid wird unter Stickstoff während 24 Stunden bei Raumtemperatur gerührt und anschließend unter vermindertem Druck zur Trockene eingedampft. Eine Lösung des Rückstandes in Methylenchlorid wird mit eiskalter verdünnter Salzsäure extrahiert. Der saure Extrakt wird mit eiskalter verdünnter Natriumcarbonatlösung alkalisch gestellt und mit Äther ausgeschüttelt. Der ätherische Auszug wird über wasserfreiem Natriumsulfat getrocknet und unter vermindertem Druck zur Trockene eingedampft. Reinigung des Rückstandes durch Säulenchromatographie an 20 g Kieselgel unter Eluieren mit 20% Äther in Methylenchlorid und anschließend mit 10% Methanol in Methylenchlorid liefert ein bernsteinfarbenes Öl. Man nimmt dieses Öl in einem Überschuß an methanolischer Salzsäure auf und dampft unter vermindertem Druck zur Trockene ein. Nach Kristallisieren des Rückstandes aus Isopropanol und Umkristallisieren aus Isopropanol erhält man 8-Chlor-5-(N-methyl-1-amino-2-propyn-3-yl)-1-phenyl-3H-2-benzazepin-dihydrochlorid als bräunliche Kristalle vom Schmelzpunkt 169—175° (Zersetzung).

Beispiel 32

Eine Mischung aus 5 g (11,4 mMol) 8-Chlor-5-(1-phthalimido-2-propin-3-yl)-1-phenyl-3H-2-benzazepin, 100 ml Äthanol und 20 ml 40prozentigem Methylamin wird während 1 Stunde bei Raumtemperatur gerührt, anschließend auf Eiswasser gegossen und mit Äther extrahiert. Die ätherische Phase wird über wasserfreiem Natriumsulfat getrocknet und unter vermindertem Druck zur Trockene eingedampft. Man nimmt den Rückstand in einem Überschuß an methanolischer Salzsäure auf und dampft unter vermindertem Druck zur Trockene ein. Durch Kristallisieren des Rückstandes aus Äthanol/Isopropanol erhält man einen bräunlichen Festkörper vom Schmelzpunkt 247—248°. Umkristallisieren aus Äthanol/Äther liefert 8-Chlor-5-(1-phthalimido-2-propin-3-yl)-1-phenyl-3H-2-benzazepin-dihydrochlorid vom Schmelzpunkt 247—248°.

Beispiel 33

Eine Mischung aus 1,3 g (2,9 mMol) 8-Chlor-5-(1-phthalimido-2-propin-3-yl)-1-(2-fluorphenyl)-3H-2-benzazepin, 50 ml Äthanol und 10 ml 40prozentigem wäßrigem Methylamin wird während 1 Stunde bei Raumtemperatur gerührt, anschließend auf Eiswasser gegossen und mit Äther extrahiert. Die ätherische Phase wird über wasserfreiem Natriumsulfat getrocknet und unter vermindertem Druck zur Trockene eingedampft. Man nimmt den Rückstand in einem Überschuß an äthanolischer Salzsäure auf, versetzt mit Äther und filtriert den erhaltenen weißlichen Festkörper ab, der einen Schmelzpunkt

von 211 – 216° hat. Durch Umkristallisieren aus Methanol/Äther erhält man 8-Chlor-5-(1-amino-2-propin-3-yl)-1-(2-fluorphenyl)-3H-2-benzazepin-dihydrochlorid als graue Nadeln vom Schmelzpunkt 216 – 218° (Zersetzung).

Beispiel 34

Eine Mischung aus 76 g (1,1 Mol) Natriumnitrit und 450 ml Schwefelsäure wird auf dem Dampfbad auf ca. 80° erwärmt bis vollständige Lösung eintritt. Nach Abkühlen auf 30° werden 232 g (1,0 Mol) 2-Amino-5-chlorbenzophenon unter Rühren portionenweise zugegeben, so daß die Temperatur zwischen 30 und 40° bleibt. Nach einer Stunde wird die Mischung langsam zu 3 Liter Eiswasser gegeben und über Hy-Flo filtriert. Das Filtrat wird unter Rühren langsam mit einer Lösung von 200 g (1,83 Mol) Natriumtetrafluoroborat in 800 ml Wasser versetzt. Der dabei entstehende Niederschlag wird abfiltriert und 2mal mit je 100 ml Wasser gewaschen.

Das feuchte 2-Benzoyl-4-chlorbenzoldiazoniumtetrafluoroborat wird in 3 l Wasser suspendiert und tropfenweise mit einer Lösung von 332 g (2 Mol) Kaliumjodid in 1 l Wasser versetzt. Die Mischung wird während 4 Stunden bei Raumtemperatur gerührt; anschließend filtriert man das Rohprodukt ab und gibt es zu 1 l siedendem Äther. Nach Filtrieren und Trocknen über Natriumsulfat wird die ätherische Lösung auf 500 ml eingeengt. Durch Zugeben von 100 ml Petroläther erhält man 5-Chlor-2-jod-benzophenon. Eine Probe des Materials wird aus Äther/Petroläther umkristallisiert und ergibt feine gelbe Prismen vom Schmelzpunkt 80 – 82°.

Beispiel 35

Die Herstellung von 5-Chlor-2'-fluor-2-jod-benzophenon erfolgt auf die gleiche Weise wie die Herstellung von 5-Chlor-2-jod-benzophenon und ergibt hellgelbe Prismen vom Schmelzpunkt 78 – 81°.

Beispiel 36

Die Herstellung von 2',5-Dichlor-2-jod-benzophenon erfolgt auf die gleiche Weise wie die Herstellung von 5-Chlor-2-jod-benzophenon und ergibt hellgelbe Prismen von Schmelzpunkt 64 – 66°.

Beispiel 37

Die Herstellung von 2'-Chlor-2-jod-benzophenon erfolgt auf die gleiche Weise wie die Herstellung von 5-Chlor-2-jod-benzophenon und ergibt schwach gelbe Prismen vom Schmelzpunkt 62 – 64°.

Beispiel 38

Eine Mischung aus 0,71 g (4,0 mMol) Palladiumchlorid, 2,1 g (8,0 mMol) Triphenylphosphin, 0,80 g (4,2 mMol) Kupfer(I)jodid, 68,8 g (0,21 Mol) 5-Chlor-2-jod-benzophenon, 200 ml Diäthylamin und 400 ml Methylenchlorid wird bei Raumtemperatur unter Argon gerührt bis alles in Lösung ist. In einer Portion gibt man 40,0 g (0,22 Mol) N-Propargyl-phthalimid hinzu und rührt die entstehende Mischung während 20 Stunden. Nach Entfernen der flüchtigen Anteile im Vakuum, wird derRückstand mit 200 ml Isopropanol verrieben. Durch Abfiltrieren des entstandenen Niederschlages erhält man rohes 1-[4-Chlor-2-benzoylphenyl]-3-phthalimidopropin. Umkristallisieren aus Aceton liefert crèmefarbene Prismen vom Schmelzpunkt 148 – 150°.

Beispiel 39

Die Herstellung von 1-[4-Chlor-2-(2-fluorbenzoyl)-phenyl]-3-phthalimidopropin erfolgt auf die gleiche Weise wie die Herstellung von 1-[4-Chlor-2-benzoylphenyl]-3-phthalimidopropin und liefert crèmefarbene Prismen vom Schmelzpunkt 158 – 161°.

Beispiel 40

Die Herstellung von 1-[4-Chlor-2-(2-chlorbenzoyl)-phenyl]-3-phthalimidopropin erfolgt auf die gleiche Weise wie die Herstellung von 1-[4-Chlor-2-benzoyl]-3-phthalimidopropin und liefert crèmefarbene Prismen vom Schmelzpunkt 144 – 145°.

Beispiel 41

Die Herstellung von 1-[2-(2-Chlorbenzoyl)phenyl]-3-phthalimidopropin erfolgt auf die gleiche Weise wie die Herstellung von 1-[4-Chlor-2-benzoylphenyl]-3-phthalimidopropin und liefert crèmefarbene Prismen vom Schmelzpunkt 149—150°.

Beispiel 42

Verfahren A

Eine Mischung aus 72 g (0,18 Mol) 1-[4-Chlor-2-benzoylphenyl]-3-phthalimidopropin, 90 ml 40prozentigem wäßrigem Methylamin und 300 ml Äthanol wird bei Raumtemperatur während 90 Minuten gerührt. Nach Verdünnen der Mischung mit 300 ml Äther wird der Niederschlag abfiltriert. Das Filtrat wird mit weiteren 300 ml Äther verdünnt, mit Wasser gewaschen und über wasserfreiem Natriumsulfat getrocknet. Eindampfen der ätherischen Lösung unter vermindertem Druck liefert ein braunes Öl, welches nach Zerreiben mit Äther einen gelben Festkörper liefert. Durch Umkristallisieren aus Äther erhält man 3-Amino-1-[2-benzoyl-4-chlorphenyl]propin als hellgelbe Prismen vom Schmelzpunkt 68—69°.

Verfahren B

Eine Mischung aus 4 g (10 mMol) 1-[4-Chlor-2-benzoylphenyl]-3-phthalimidopropin, 0,6 g (16 mMol) 85prozentigem Hydrazinhydrat und 150 ml 95prozentigem Äthanol wird während 5,5 Stunden unter Rückfluß zum Sieden erhitzt. Nach dem Abkühlen werden unlösliche Anteile durch Filtration entfernt. Das Filtrat wird mit Wasser verdünnt, mit Salzsäure angesäuert und mit Äther ausgeschüttelt. Die wäßrige Phase wird mit verdünnter Natriumcarbonatlösung basisch gestellt und mit Methylenchlorid extrahiert. Die Methylenchloridphase wird über wasserfreiem Natriumsulfat getrocknet und unter vermindertem Druck zur Trockene eingedampft. Durch Umkristallisieren des Rückstandes aus Äther/Petroläther erhält man 3-Amino-1-[2-benzoyl-4-chlorphenyl]propin als hellgelben Festkörper vom Schmelzpunkt 68—69°, welcher in allen Belangen identisch mit authentischem Material ist.

Durch Behandeln einer Lösung von 3-Amino-1-[4-chlor-2-benzoylphenyl]propion in Methanol mit 6prozentiger methanolischer Salzsäure und Ausfällen des Produktes mit Äther erhält man das entsprechende rohe Hydrochlorid. Durch Umkristallisieren aus Methanol/Äther erhält man 3-Amino-1-[2-benzoyl-4-chlorphenyl]propin-hydrochlorid als weiße Nadeln vom Schmelzpunkt 173—174°.

Beispiel 43

Verfahren A

In Analogie zur Herstellung von 3-Amino-1-[4-chlor-2-benzoylphenyl]propin (Verfahren A) erhält man 3-Amino-1-[4-chlor-2-(2-fluorbenzoyl)phenyl]propin als gelbe Prismen vom Schmelzpunkt 89—91°.

Verfahren B

Eine Mischung aus 50 g 1-[4-Chlor-2-(2-fluorbenzoyl)phenyl]-3-phthalimidopropin, 50 ml 40prozentigem wäßrigem Methylamin und 150 ml Dimethylformamid wird während 25 Minuten bei Raumtemperatur gerührt. Anschließend versetzt man tropfenweise mit 500 ml Wasser und filtriert den entstandenen Niederschlag ab. Eine Lösung des Niederschlages in Methylenchlorid wird über wasserfreiem Natriumsulfat getrocknet und unter vermindertem Druck zu einem hellgelben Festkörper eingedampft. Durch Umkristallisieren aus Äther erhält man 3-Amino-1-[4-chlor-2-(2-fluorbenzoyl)phenyl]propin als hellgelbe Prismen vom Schmelzpunkt 89—91°. Dieses Material ist in jeder Hinsicht identisch mit authentischem Material.

Verfahren C

Eine Mischung aus 400 g (0,96 Mol) 1-[4-Chlor-2-(2-fluorbenzoyl)phenyl]-3-phthalimidopropin, 1,3 l Äthanol und 300 ml 40prozentigem wäßrigem Methylamin wird bei Raumtemperatur während 2 Stunden gerührt. Man versetzt anschließend tropfenweise mit 2,8 l Wasser und filtriert den entstandenen Niederschlag ab, wobei man einen hellgelben Festkörper vom Schmelzpunkt 79—80° erhält. Durch

Umkristallisieren aus Äther erhält man 3-Amino-1-[4-chlor-2-(2-fluorbenzoyl)phenyl]propin als hellgelbe Prismen vom Schmelzpunkt 89—91°. Dieses Material ist in jeder Hinsicht identisch mit authentischem Material.

Beispiel 44

In Analogie zur Herstellung von 3-Amino-1-[4-chlor-2-(2-chlorbenzoyl)phenyl]propin (Verfahren A) erhält man 3-Amino-1-[4-chlor-2-(2-chlorbenzoyl)phenyl]propin als hellgelbe Prismen vom Schmelzpunkt 81—82°.

Beispiel 45

In Analogie zur Herstellung von 3-Amino-1-[4-chlor-2-benzoylphenyl]propin (Verfahren A) erhält man 3-Amino-1-[2-(2-chlorbenzoyl)phenyl]propin als bernsteinfarbenes Öl.

Das Hydrochlorid von 3-Amino-1-[2-(2-chlorbenzoyl)-phenyl]propin erhält man, indem man eine Lösung dieses Materials in Methanol mit einem Überschuß an 6prozentiger methanolischer Salzsäure versetzt und das Produkt durch Zugabe von Äther ausfällt. Durch Umkristallisieren aus Methanol/Äther erhält man 3-Amino-1-[2-(2-chlorbenzoyl)-phenyl]propin-hydrochlorid als weiße Nadeln vom Schmelzpunkt 160—162°

Beispiel 46

Herstellung von Tabletten (Naßgranulierung)

| Bestandteile | mg/Tablette | mg/Tablette | mg/Tablette | mg/Tablette |
|---|---|---|---|---|
| 1. 8-Chlor-1-phenyl-3H-2-benzazepin-2-oxid oder<br>3-[8-Chlor-1-(2-fluorphenyl)-3H-2-benzazepin-5-yl]-2-propin-1-amin | 1 | 5 | 10 | 50 |
| 2. Lactose | 195 | 230 | 264 | 263 |
| 3. Modifizierte Stärke | 12,5 | 15 | 17,5 | 20 |
| 4. Vorgelatinisierte Stärke | 12,5 | 15 | 17,5 | 20 |
| 5. Kornstärke | 25 | 30 | 35 | 40 |
| 6. Magnesiumstearat | 4 | 5 | 6 | 7 |
| 7. Destilliertes Wasser q. s. | — | — | — | — |
| Gesamtgewicht | 250 mg | 300 mg | 350 mg | 400 mg |

Verfahren

Man mischt die Bestandteile 1—5 in einem geeigneten Mixer, granuliert mit genügend destilliertem Wasser zu geeigneter Konsistenz, mahlt und trocknet in einem geeigneten Ofen. Man mahlt erneut und vermischt mit Magnesiumstearat. Anschließend wird auf einer geeigneten Presse zu Tabletten entsprechender Größe verpreßt.

Beispiel 47

Herstellung von Tabletten (Direkte Verpressung)

| Bestandteile | mg/Tablette | mg/Tablette | mg/Tablette | mg/Tablette |
|---|---|---|---|---|
| 1. 8-Chlor-1-phenyl-3H-1-benzazepin-2-oxid oder<br>3-[8-Chlor-1-(2-fluorphenyl)-3H-2-benzazepin-5-yl]-2-propin-1-amin | 1 | 5 | 10 | 50 |
| 2. Lactose | 127 | 142,5 | 182 | 206 |
| 3. Microkristalline Zellulose | 40 | 50 | 60 | 80 |
| 4. Stärke für die direkte Verpressung | 10 | 12 | 15 | 20 |
| 5. Kornstärke | 20 | 25 | 30 | 40 |
| 6. Magnesiumstearat | 2 | 2,5 | 3 | 4 |
| Gesamtgewicht | 200 mg | 250 mg | 300 mg | 400 mg |

Verfahren

Man vermischt die Bestandteile 1—5 während 1—15 Minuten in einem geeigneten Mixer, versetzt mit dem Magnesiumstearat und mischt während 5 Minuten. Anschließend verpreßt man auf einer geeigneten Presse zu Tabletten entsprechender Größe.

Beispiel 48

Herstellung von Kapseln

| Bestandteile | mg/Kapsel | mg/Kapsel | mg/Kapsel | mg/Kapsel |
|---|---|---|---|---|
| 1. 8-Chlor-1-phenyl-3H-2-benzazepin-2-oxid oder<br>3-[8-Chlor-1-(2-fluorphenyl)-3H-2-benzazepin-5-yl]-2-propin-1-amin | 1 | 5 | 10 | 50 |
| 2. Lactose | 149 | 182,5 | 215 | 250 |
| 3. Kornstärke | 40 | 50 | 60 | 80 |
| 4. Talk | 8 | 10 | 12 | 16 |
| 5. Magnesiumstearat | 2 | 2,5 | 3 | 4 |
| Kapselfüllgewicht | 200 mg | 250 mg | 300 mg | 400 mg |

Verfahren

Man vermischt die Bestandteile 1, 2 und 3 in einem geeigneten Mixer, mahlt mit den Bestandteilen 4 und 5 und mischt gut. Anschließend wird auf einer geeigneten Maschine in Kapseln entsprechender Größe abgefüllt.

**Patentansprüche für die Vertragsstaaten: BE, CH, DE, FR, GB, IT, LI, LU, NL, SE**

1. 3H-2-Benzazepine der allgemeinen Formel

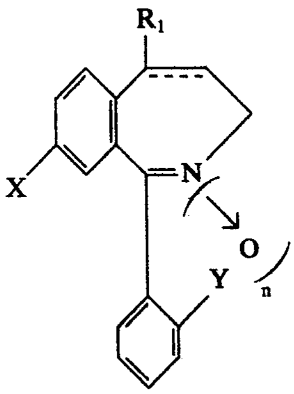

(I)

worin X Wasserstoff, Chlor oder Brom, Y Wasserstoff, Fluor oder Chlor, die gestrichelte Linie eine fakultative Bindung, n die Zahl 0 oder 1, $R_1$ Wasserstoff, Brom, Chlor, Jod oder ein Rest der Formel

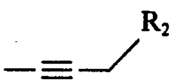 oder 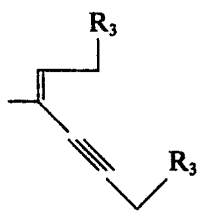

und $R_2$ Wasserstoff, $(C_1-C_7)$-Alkyl, Hydroxy, Amino, Mono-$(C_1-C_7)$-alkylamino oder Di-$(C_1-C_7)$-alkylamino und $R_3$ Hydroxy oder Amino bedeuten, mit der Maßgabe, daß X und Y nicht beide Wasserstoff bedeuten, und daß die gestrichelte Linie eine zusätzliche Bindung bedeutet, wenn $R_1$ nicht Wasserstoff bedeutet,
und pharmazeutisch annehmbare Salze davon.

2. Verbindungen gemäß Anspruch 1, gekennzeichnet durch die allgemeine Formel

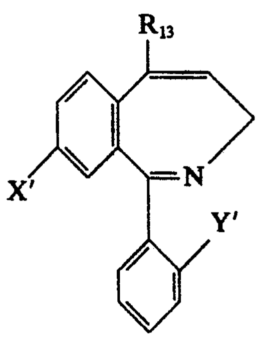

(Ie)

worin X' Chlor, Y' Wasserstoff, Fluor oder Chlor, $R_{13}$ ein Rest der Formel

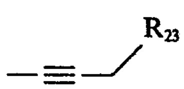 oder 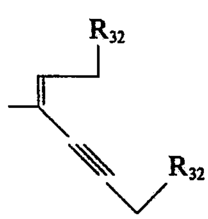

und $R_{23}$ Amino, Mono-$(C_1-C_7)$-alkylamino oder Di-$(C_1-C_7)$-alkylamino und $R_{32}$ Amino bedeuten.

3. Verbindungen gemäß Anspruch 2, dadurch gekennzeichnet, daß $R_{13}$ ein Rest der Formel

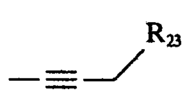

bedeutet und $R_{23}$ die in Anspruch 2 angegebene Bedeutung besitzt.

4. 8-Chlor-5-(1-amino-2-propin-3-yl)-1-(2-fluorphenyl)-3H-2-benzazepin.
5. 8-Chlor-5-(1-amino-2-propin-3-yl)-1-phenyl-3H-2-benzazepin.
6. 8-Chlor-5-(1-dimethylamino-2-propin-3-yl)-1-(2-fluorphenyl)-3H-2-benzazepin.
7. 8-Chlor-1-phenyl-3H-2-benzazepin-2-oxid.
8. Verbindungen der allgemeinen Formel

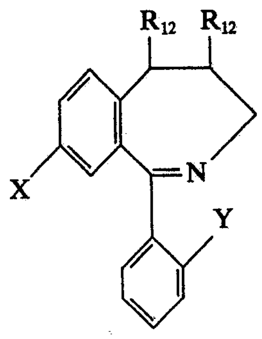

(III)

worin X Wasserstoff, Chlor oder Brom und Y Wasserstoff, Fluor oder Chlor bedeuten, mit der Maßgabe, daß X und Y nicht beide Wasserstoff bedeuten, und $R_{12}$ Chlor, Brom oder Jod bedeutet.

9. Verbindungen der allgemeinen Formel

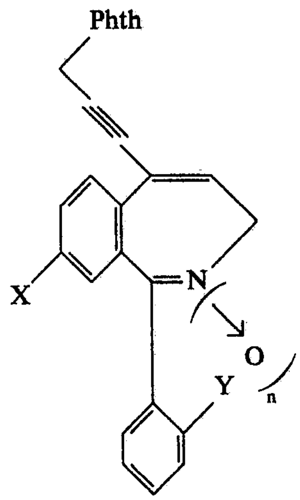

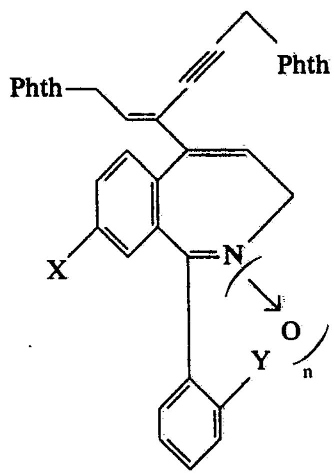

(V) oder (VI)

worin X Wasserstoff, Chlor oder Brom und Y Wasserstoff, Fluor oder Chlor bedeuten, mit der Maßgabe, daß X und Y nicht beide Wasserstoff bedeuten, und n die Zahl 0 oder 1 und Phth Phthalimido bedeuten.

10. Verbindungen gemäß einem der Ansprüche 1—7 als pharmazeutische Wirkstoffe.
11. Verbindungen gemäß einem der Ansprüche 1—7 als Sedativa und Anxiolytika.
12. Verbindungen gemäß einem der Ansprüche 2—6 als Antidepressiva.
13. Verfahren zur Herstellung von Verbindungen gemäß einem der Ansprüche 1—7, dadurch gekennzeichnet, daß man

a) eine Verbindung der allgemeinen Formel

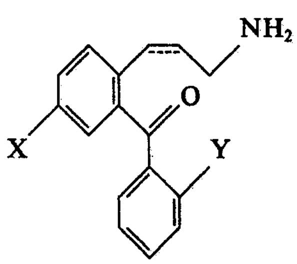

(II)

worin X und Y die in Anspruch 1 angegebene Bedeutung besitzen und die gestrichelte Linie eine

fakultative Bindung bedeutet, cyclisiert, oder

b) eine Verbindung der allgemeinen Formel

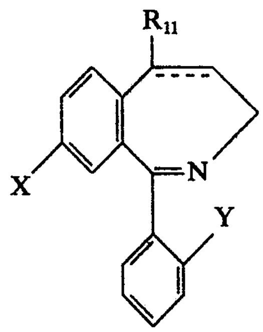

(Ia)

worin X und Y die in Anspruch 1 angegebene Bedeutung besitzen, $R_{11}$ Wasserstoff, Brom, Chlor oder Jod und die gestrichelte Linie eine fakultative Bindung bedeuten, mit der Maßgabe, daß die gestrichelte Linie eine zusätzliche Bindung bedeutet, wenn $R_{11}$ nicht Wasserstoff bedeutet, oxidiert, oder

c) eine Verbindung der allgemeinen Formel

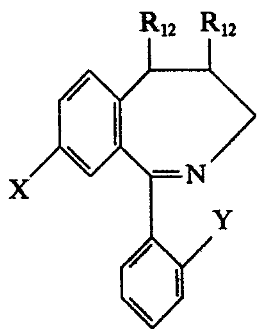

(III)

worin X und Y die in Anspruch 1 angegebene Bedeutung besitzen und $R_{12}$ Chlor, Brom oder Jod bedeutet,
dehydrohalogeniert, oder

d) eine Verbindung der allgemeinen Formel

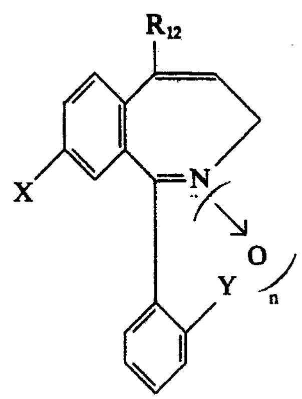

(Ib)

worin X, Y und n die in Anspruch 1 angegebene und $R_{12}$ obige Bedeutung besitzen, in Gegenwart von einem Palladiumsalz, Kupfer(I)jodid, einem Organophosphin und einem sekundären oder tertiären Amin mit einer Verbindung der allgemeinen Formel

$$HC \equiv C-CH_2-R_{21} \qquad (IV)$$

worin $R_{21}$, Wasserstoff, $(C_1-C_7)$-Alkyl, Hydroxy, Mono-$(C_1-C_7)$-alkylamino oder Di-$(C_1-C_7)$-alkylamino bedeutet,
umsetzt, oder

e) in einer Verbindung der allgemeinen Formel

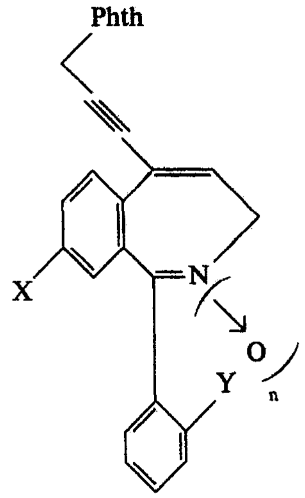

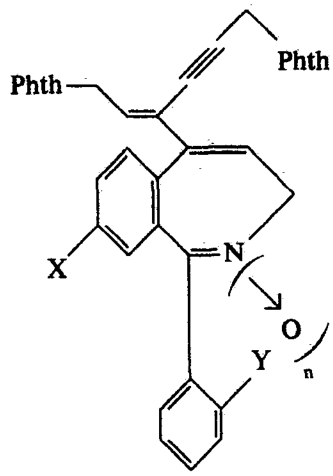

(V) oder (VI)

worin X, Y und n die in Anspruch 1 angegebene Bedeutung besitzen und Phth Phthalimido bedeutet,
die Phthaloylgruppe(n) abspaltet und erwünschtenfalls

f) eine erhaltene Verbindung der allgemeinen Formel I in ein pharmazeutisch annehmbares Salz überführt.

14. Arzneimittel, enthaltend eine Verbindung gemäß einem der Ansprüche 1—7.

15. Sedativ und anxiolytisch wirksame Mittel, enthaltend eine Verbindung gemäß einem der Ansprüche 1—7.

16. Antidepressiv wirksame Mittel, enthaltend eine Verbindung gemäß einem der Ansprüche 2—6.

**Patentansprüche für den Vertragsstaat: AT**

1. Verfahren zur Herstellung von 3H-2-Benzazepinen der allgemeinen Formel

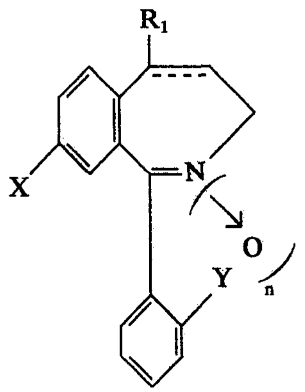

(I)

worin X Wasserstoff, Chlor oder Brom, Y Wasserstoff, Fluor oder Chlor, die gestrichelte Linie eine fakultative Bindung, n die Zahl 0 oder 1, $R_1$ Wasserstoff, Brom, Chlor, Jod oder ein Rest der Formel

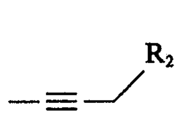

oder

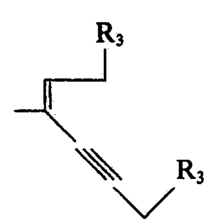

und $R_2$ Wasserstoff, $(C_1-C_7)$-Alkyl, Hydroxy, Amino, Mono-$(C_1-C_7)$-alkylamino oder Di-$(C_1-C_7)$-alkylamino und $R_3$ Hydroxy oder Amino bedeuten, mit der Maßgabe, daß X und Y nicht beide Wasserstoff bedeuten, und daß die gestrichelte Linie eine zusätzliche Bindung bedeutet, wenn $R_1$ nicht Wasserstoff bedeutet,
und von pharmazeutisch annehmbaren Salzen davon, dadurch gekennzeichnet, daß man

a) eine Verbindung der allgemeinen Formeln

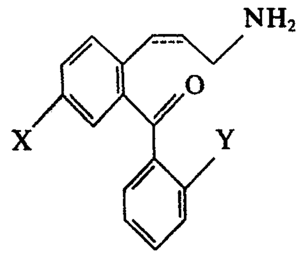

(II)

worin X und Y obige Bedeutung besitzen und die gestrichelte Linie eine fakultative Bindung bedeutet,

cyclisiert, oder

b) eine Verbindung der allgemeinen Formel

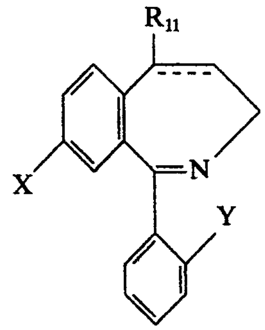

(Ia)

worin X und Y obige Bedeutung besitzen, $R_{11}$ Wasserstoff, Brom, Chlor oder Jod und die gestrichelte Linie eine fakultative Bindung bedeuten, mit der Maßgabe, daß die gestrichelte Linie eine zusätzliche Bindung bedeutet, wenn $R_{11}$ nicht Wasserstoff bedeutet,

oxidiert, oder

c) eine Verbindung der allgemeinen Formel

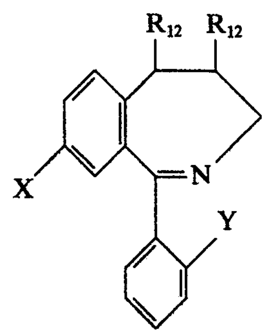

(III)

worin X und Y obige Bedeutung besitzen und $R_{12}$ Chlor, Brom oder Jod bedeutet,

dehydrohalogeniert, oder

d) eine Verbindung der allgemeinen Formel

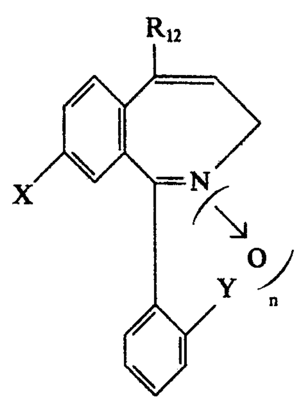

(Ib)

worin X, Y, n und $R_{12}$ obige Bedeutung besitzen, in Gegenwart von einem Palladiumsalz, Kupfer(I)jodid, einem Organophosphin und einem sekundären oder tertiären Amin mit einer Verbindung der allgemeinen Formel

$$HC \equiv C-CH_2-R_{21} \qquad (IV)$$

worin $R_{21}$ Wasserstoff, $(C_1-C_7)$-Alkyl, Hydroxy, Mono-$(C_1-C_7)$-alkylamino oder Di-$(C_1-C_7)$-alkylamino bedeutet, umsetzt, oder

e) in einer Verbindung der allgemeinen Formel

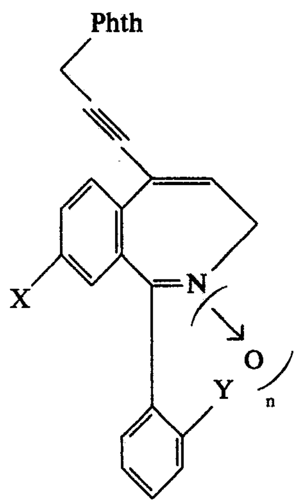

(V)

oder

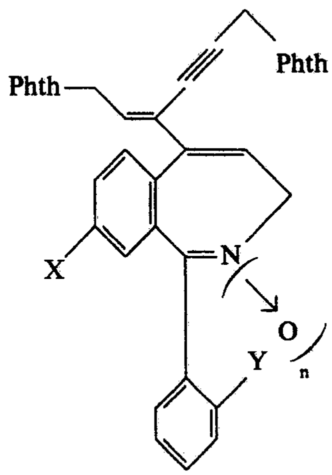

(VI)

worin X, Y und n obige Bedeutung besitzen und Phth Phthalimido bedeutet, die Phthaloylgruppe(n) abspaltet und erwünschtenfalls

f) eine erhaltene Verbindung der allgemeinen Formel I in ein pharmazeutisch annehmbares Salz überführt.

2. Verfahren gemäß Anspruch 1, dadurch gekennzeichnet, daß man eine Verbindung der allgemeinen Formel

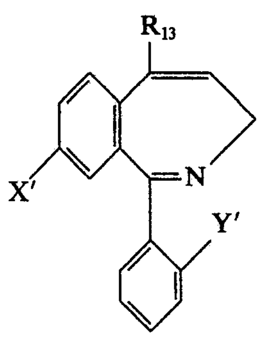

(Ie)

worin X′ Chlor, Y′ Wasserstoff, Fluor oder Chlor, $R_{13}$ ein Rest der Formel

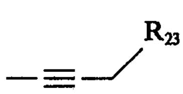

oder

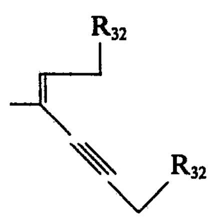

und $R_{23}$ Amino, Mono-$(C_1-C_7)$-alkylamino oder Di-$(C_1-C_7)$-alkylamino und $R_{32}$ Amino bedeuten, herstellt.

3. Verfahren gemäß Anspruch 2, dadurch gekennzeichnet, daß man eine Verbindung der in Anspruch 2 definierten allgemeinen Formel Ie herstellt, worin $R_{13}$ einen Rest der Formel

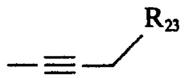

bedeutet und $R_{23}$ die in Anspruch 2 angegebene Bedeutung besitzt.

4. Verfahren gemäß Anspruch 3, dadurch gekennzeichnet, daß man 8-Chlor-5-(1-amino-2-propin-3-yl)-1-(2-fluorphenyl)-3H-2-benzazepin herstellt.

5. Verfahren gemäß Anspruch 3, dadurch gekennzeichnet, daß man 8-Chlor-5-(1-amino-2-propin-3-yl)-1-phenyl-3H-2-benzazepin herstellt.

6. Verfahren gemäß Anspruch 3, dadurch gekennzeichnet, daß man 8-Chlor-5-(1-dimethylamino-2-propin-3-yl)-1-(2-fluorphenyl)-3H-2-benzazepin herstellt.

7. Verfahren gemäß Anspruch 1, dadurch gekennzeichnet, daß man 8-Chlor-1-phenyl-3H-2-benzazepin-2-oxid herstellt.

**Claims for the Contracting States: BE, CH, DE, FR, GB, IT, LI, LU, NL, SE**

1. 3H-2-Benzazepines of the general formula

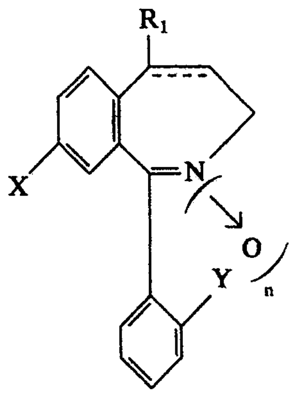

(I)

wherein X signifies hydrogen, chlorine or bromine, Y signifies hydrogen, fluorine or chlorine, the dotted line signifies an optional bond, n signifies the number 0 or 1, $R_1$ signifies hydrogen, bromine, chlorine, iodine or a residue of the formula

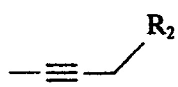

or

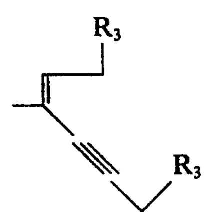

and $R_2$ signifies hydrogen, $(C_1-C_7)$-alkyl, hydroxy, amino, $(C_1-C_7)$-alkylamino or di-$(C_1-C_7)$-alkylamino and $R_3$ signifies hydroxy or amino, with the proviso that X and Y do not both signify hydrogen, and that the dotted line signifies an additional bond when $R_1$ does not signify hydrogen, and pharmaceutically acceptable salts thereof.

2. Compounds according to claim 1, characterized by the general formula

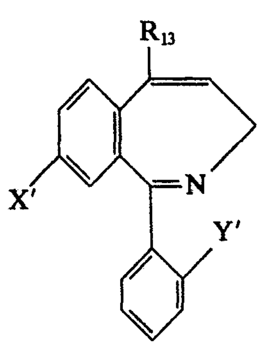

(I e)

wherein X′ signifies chlorine, Y′ signifies hydrogen, fluorine or chlorine, $R_{13}$ signifies a residue of the

formula

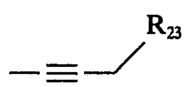

or

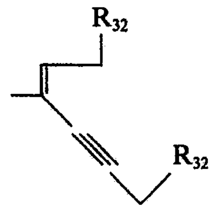

and $R_{23}$ signifies amino, mono-($C_1-C_7$)-alkylamino or di-($C_1-C_7$)-alkylamino and $R_{32}$ signifies amino.

3. Compounds according to claim 2, characterized in that $R_{13}$ signifies a residue of the formula

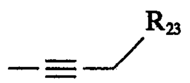

and $R_{23}$ has the significance given in claim 2.

4. 8-Chloro-5-(1-amino-2-propyn-3-yl)-1-(2-fluorophenyl)-3H-2-benzazepine.

5. 8-Chloro-5-(1-amino-2-propyn-3-yl)-1-phenyl-3H-2-benzazepine.

6. 8-Chloro-5-(1-dimethylamino-2-propyn-3-yl)-1-(2-fluorophenyl)-3H-2-benzazepine.

7. 8-Chloro-1-phenyl-3H-2-benzazepine-2-oxide.

8. Compounds of the general formula

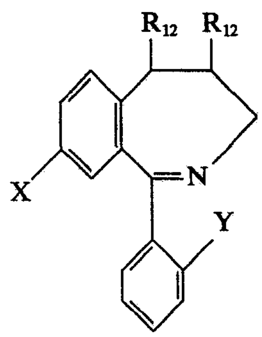

(III)

wherein X signifies hydrogen, chlorine or bromine and Y signifies hydrogen, fluorine or chlorine, with the proviso that X and Y do not both signify hydrogen, and $R_{12}$ signifies chlorine, bromine or iodine.

9. Compounds of the general formulae

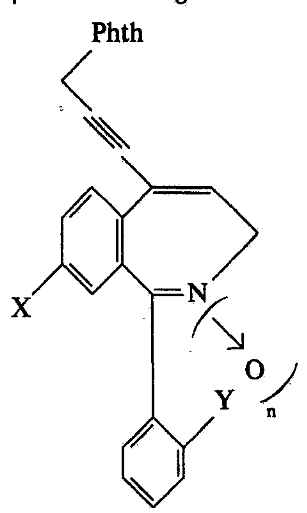

(V)

or

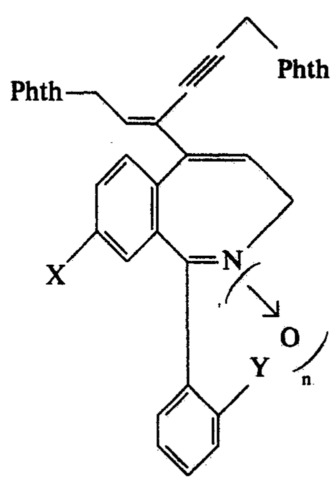

(VI)

wherein X signifies hydrogen, chlorine or bromine and Y signifies hydrogen, fluorine or chlorine, with the proviso that X and Y do not both signify hydrogen, and n signifies the number 0 or 1 and Phth signifies phthalimido.

10. Compounds according to any one of claims 1—7 as pharmaceutically active substances.

11. Compounds according to any one of claims 1—7 as sedatives and anxiolytics.

12. Compounds according to any one of claims 2—6 as antidepressants.

13. A process for the manufacture of compounds according to any one of claims 1—7, characterized by

a) cyclizing a compound of the general formula

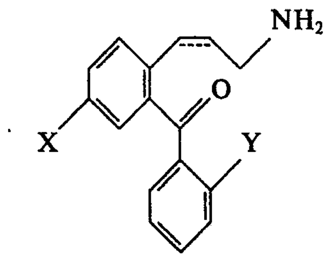

(II)

wherein X and Y have the significance given in claim 1 and the dotted line signifies an optional bond,
or

b) oxidizing a compound of the general formula

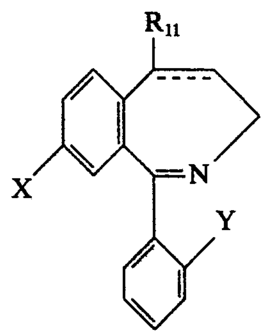

(Ia)

wherein X and Y have the significance given in claim 1, $R_{11}$ signifies hydrogen, bromine, chlorine or iodine and the dotted line signifies an optional bond, with the proviso that the dotted line signifies an additional bond when $R_{11}$ does not signify hydrogen,
or

c) dehydrohalogenating a compound of the general formula

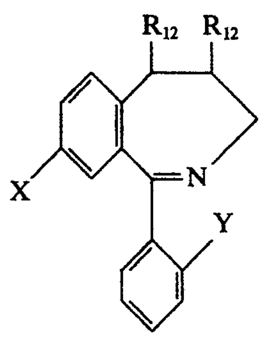

(III)

wherein X and Y have the significance given in claim 1 and $R_{12}$ signifies chlorine, bromine or iodine,
or

d) reacting a compound of the general formula

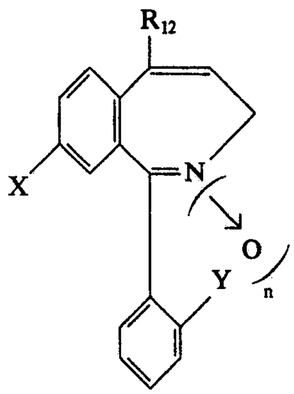

(Ib)

wherein X, Y and n have the significance given in claim 1 and $R_{12}$ has the above significance, with a compound of the general formula

$$HC{\equiv}C-CH_2-R_{21} \qquad (IV)$$

wherein $R_{21}$ signifies hydrogen, $(C_1-C_7)$-alkyl, hydroxy, mono-$(C_1-C_7)$-alkylamino or di-$(C_1-C_7)$-alkylamino,
in the presence of a palladium salt, copper (I) iodide, an organophosphine and a secondary or tertiary amine, or

e) cleaving off the phthaloyl group(s) in a compound of the general formula

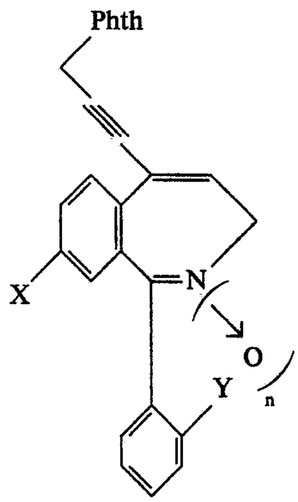

(V) or

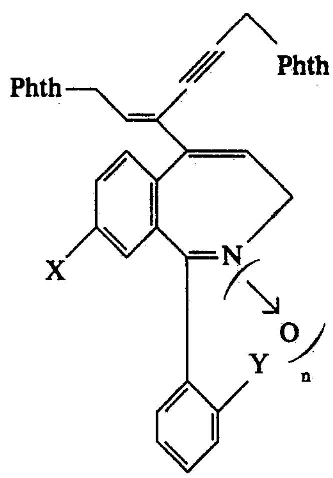

(VI)

wherein X, Y and n have the significance given in claim 1 and Phth signifies phthalimido, and, if desired,

f) converting a compound of general formula I obtained into a pharmaceutically acceptable salt.

14. A medicament containing a compound according to any one of claims 1—7.
15. A sedative and anxiolytic medicament containing a compound according to any one of claims 1—7.
16. An antidepressant medicament containing a compound according to any one of claims 2—6.

**Claims for the Contracting State: AT**

1. A process for the manufacture of 3H-2-benzazepines of the general formula

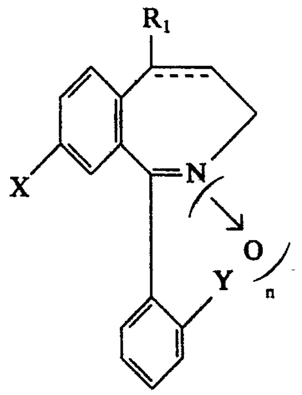

(I)

wherein X signifies hydrogen, chlorine or bromine, Y signifies hydrogen, fluorine or chlorine, the dotted line signifies an optional bond, n signifies the number 0 or 1, $R_1$ signifies hydrogen, bromine, chlorine, iodine or a residue of the formula

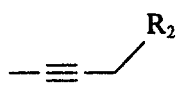

or

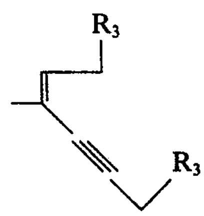

and $R_2$ signifies hydrogen, $(C_1-C_7)$-alkyl, hydroxy, amino, mono-$(C_1-C_7)$-alkylamino or di-$(C_1-C_7)$-alkylamino and $R_3$ signifies hydroxy or amino, with the proviso that X and Y do not both signify hydrogen, and that the dotted line signifies an additional bond when $R_1$ does not signify hydrogen,
and of pharmaceutically acceptable salts thereof, characterized by

a) cyclizing a compound of the general formula

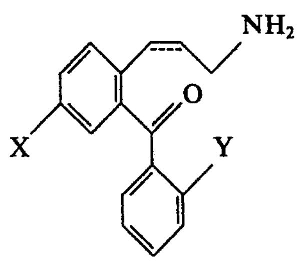

(II)

wherein X and Y have the above significance and the dotted line signifies an optional bond,
or

b) oxidizing a compound of the general formula

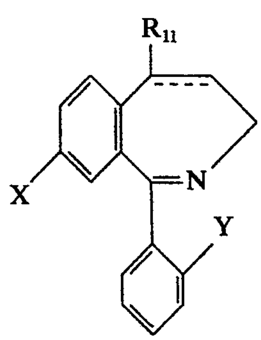

(Ia)

wherein X and Y have the above significance, $R_{11}$ signifies hydrogen, bromine, chlorine or iodine and the dotted line signifies an optional bond, with the proviso that the dotted line signifies an additional bond when $R_{11}$ does not signify hydrogen,

or

c) dehydrohalogenating a compound of the general formula

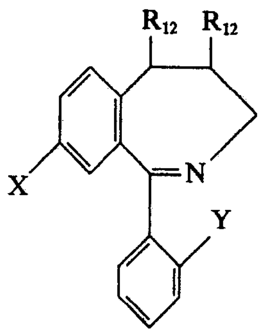

(III)

wherein X and Y have the above significance and $R_{12}$ signifies chlorine, bromine or iodine, or

d) reacting a compound of the general formula

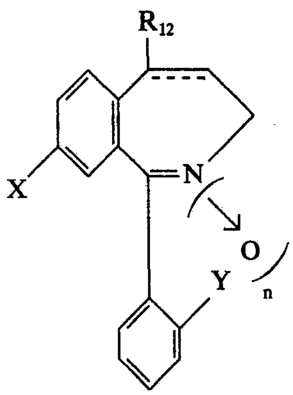

(Ib)

wherein X, Y, n and $R_{12}$ have the above significance, with a compound of the general formula

$HC \equiv C-CH_2-R_{21}$ (IV)

wherein $R_{21}$ signifies hydrogen, $(C_1-C_7)$-alkyl, hydroxy, mono-$(C_1-C_7)$-alkylamino or di-$(C_1-C_7)$-alkylamino, in the presence of a palladium salt, copper (I) iodide, an organophosphine and a secondary or tertiary amine, or

e) cleaving off the phthaloyl group(s) in a compound of the general formula

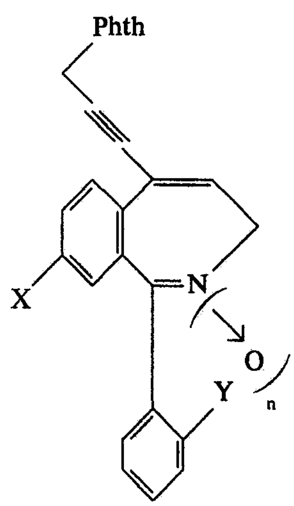

(V) or 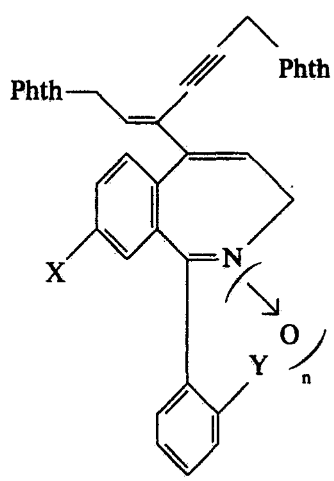

(VI)

wherein X, Y and n have the above significance and Phth signifies phthalimido, and, if desired,

f) converting a compound of general formula I obtained into a pharmaceutically acceptable salt.

2. A process according to claim 1, characterized in that a compound of the general formula

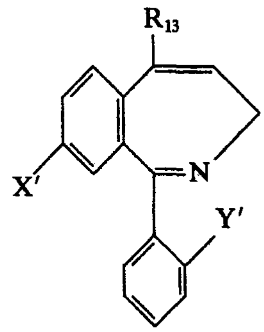

(Ie)

wherein X′ signifies chlorine, Y′ signifies hydrogen, fluorine or chlorine, $R_{13}$ signifies a residue of the formula

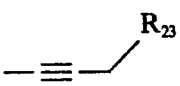

or

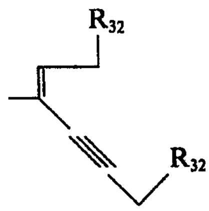

and $R_{23}$ signifies amino, mono-($C_1-C_7$)-alkylamino or di-($C_1-C_7$)-alkylamino and $R_{32}$ signifies amino is manufactured.

3. A process according to claim 2, characterized in that a compound of general formula Ie defined in claim 2 wherein $R_{13}$ signifies a residue of the formula

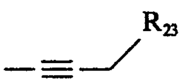

and $R_{23}$ has the significance given in claim 2 is manufactured.

4. A process according to claim 3, characterized in that 8-chloro-5-(1-amino-2-propyn-3-yl)-1-(2-fluorophenyl)-3H-2-benzazepine is manufactured.

5. A process according to claim 3, characterized in that 8-chloro-5-(1-amino-2-propyn-3-yl)-1-phenyl-3H-2-benzazepine is manufactured.

6. A process according to claim 3, characterized in that 8-chloro-5-(1-dimethylamino-2-propyn-3-yl)-1-(2-fluorophenyl)-3H-2-benzazepine is manufactured.

7. A process according to claim 1, characterized in that 8-chloro-1-phenyl-3H-2-benzazepine 2-oxide is manufactured.

**Revendications pour les Etats contractants: BE, CH, DE, FR, GB, IT, LI, LU, NL, SE**

1. 3H-2-benzazépines de formule générale:

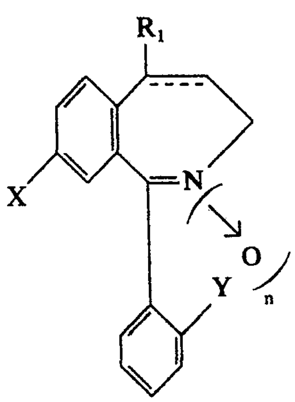

(I)

dans laquelle:

X représente l'hydrogène, le chlore ou le brome,
Y l'hydrogène, le fluor ou le chlore,
le trait interrompu une liaison facultative,
n est égal à 0 ou 1,
$R_1$ représente l'hydrogène, le brome, le chlore, l'iode ou un reste de formule:

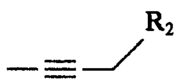 ou 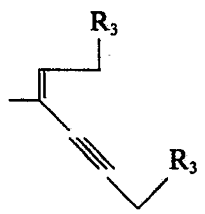

et
$R_2$ représente l'hydrogène, un groupe alkyle en $C_1-C_7$, hydroxy, amino, monoalkylamino en $C_1-C_7$ ou di-(alkyle en $C_1-C_7$)-amino et
$R_3$ représente un groupe hydroxy ou amino; étant spécifié que X et Y ne peuvent représenter tous deux l'hydrogène, et que le trait interrompu représente une liaison supplémentaire lorsque $R_1$ ne représente pas l'hydrogène,
et leurs sels acceptables pour l'usage pharmaceutique.

2. Composés selon la revendication 1, caractérisés par la formule générale:

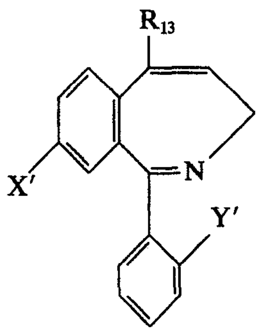

(Ie)

dans laquelle:
X' représente le chlore,
Y' l'hydrogène, le fluor ou le chlore,
$R_{13}$ un reste de formule:

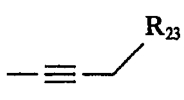 ou 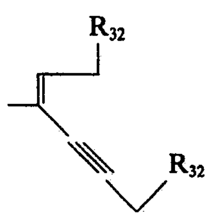

et
$R_{23}$ un groupe amino, monoalkylamino en $C_1-C_7$ ou di-(alkyle en $C_1-C_7$)-amino et
$R_{32}$ représente un groupe amino.

3. Composés selon la revendication 2, caractérisés en ce que $R_{13}$ représente un reste de formule

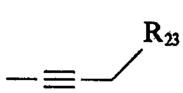

et
$R_{23}$ a la signification indiquée dans la revendication 2.

4. La 8-chloro-5-(1-amino-2-propyne-3-yl)-1-(2-fluorophényl)-3H-2-benzazépine.

5. La 8-chloro-5-(1-amino-2-propyne-3-yl)-1-phényl-3H-2-benzazépine.

6. La 8-chloro-5-(1-diméthylamino-2-propyne-3-yl)-1-(2-fluorophényl)-3H-2-benzazépine.

7. Le 2-oxyde de 8-chloro-1-phényl-3H-2-benzazépine.

8. Composés de formule générale:

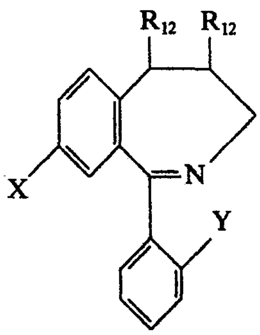

(III)

dans laquelle:
X représente l'hydrogène, le chlore ou le brome et
Y représente l'hydrogène, le flour ou le chlore, étant spécifié que X et Y ne peuvent représenter tous deux l'hydrogène, et
$R_{12}$ représente le chlore, le brome ou l'iode.

9. Composés de formules générales:

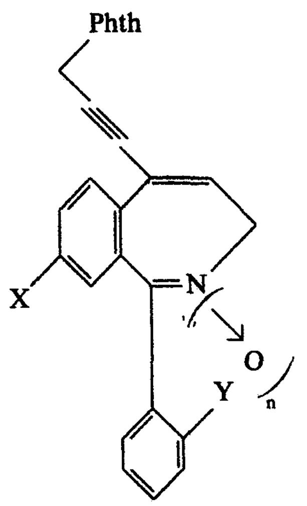

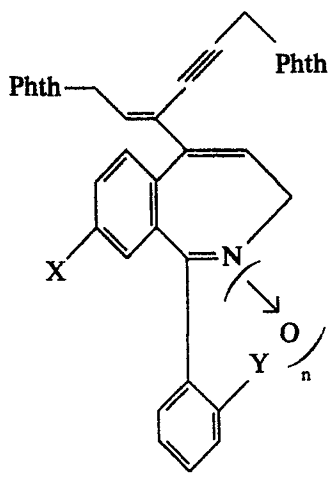

(V) ou (VI)

dans lesquelles
X représente l'hydrogène, le chlore ou le brome et
Y représente l'hydrogène, le fluor ou le chlore, étant spécifié que X et Y ne peuvent représenter tous deux l'hydrogène,
n est égal à 0 ou 1 et
Phth représente le radical phtalimido.

10. Composés selon l'une des revendications 1 à 7 en tant que substances actives pharmaceutiques.

11. Composés selon l'une des revendications 1 à 7 en tant que sédatifs et anxiolytiques.

12. Composés selon l'une des revendications 2 à 6 en tant qu'antidépressifs.

13. Procédé de préparation des composés selon l'une des revendications 1 à 7, caractérisé en ce que:

a) on cyclise un composé de formule générale

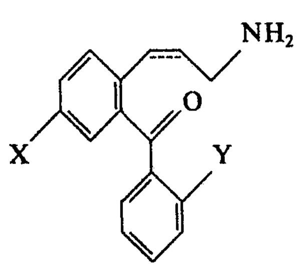

(II)

dans laquelle:
X et Y ont les significations indiquées dans la revendication 1 et
le trait interrompu représente une liaison facultative, ou bien

b) on oxyde un composé de formule générale

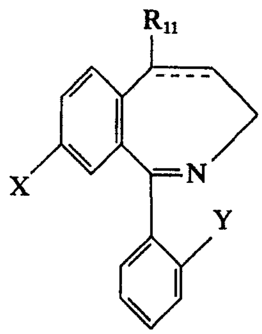

(Ia)

dans laquelle:
X et Y ont les significations indiquées dans la revendication 1,
$R_{11}$ représente l'hydrogène, le brome, le chlore ou l'iode, et
le trait interrompu représente une liaison facultative, étant spécifée que le trait interrompu représente une liaison supplémentaire lorsque $R_{11}$ ne représente pas l'hydrogène, ou bien

c) on soumet à déshydrohalogénation un composé de formule générale:

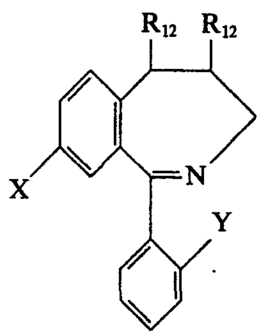

(III)

dans laquelle:
X et Y ont les significations indiquées dans la revendication 1 et
$R_{12}$ représente le chlore, le brome ou l'iode, ou bien

d) on fait réagir un composé de formule générale:

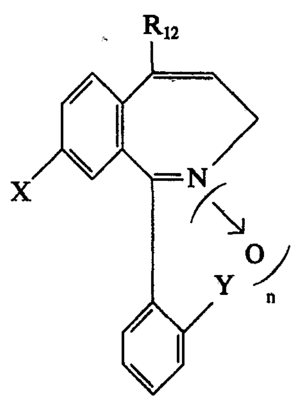

(Ib)

dans laquelle:
X, Y et n ont les significations indiquées dans la revendication 1 et
$R_{12}$ a la signification ci-dessus,
en présence d'un sel de palladium, d'iodure de cuivre-I, d'une organophosphine et d'une amine secondaire ou tertiaire, avec un composé de formule générale:

$$HC{\equiv}C-CH_2-R_{21} \qquad (IV)$$

dans laquelle:
$R_{21}$ représente l'hydrogène, un groupe alkyle en $C_1-C_7$, hydroxy, monoalkylamino en $C_1-C_7$ ou

di-(alkyle en $C_1-C_7$)-amino, ou bien

e) dans un composé de formule générale:

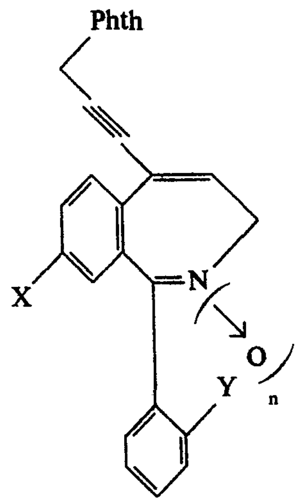

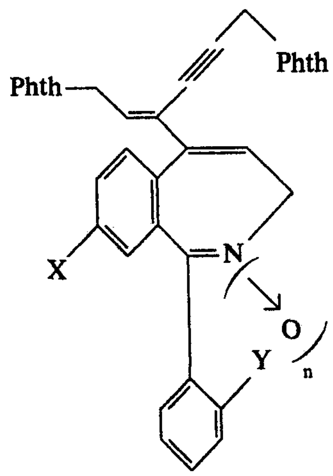

(V) ou (VI)

dans laquelle:
X, Y et n ont les significations indiquées dans la revendication 1, et
Phth représente le radical phtalimido, on élimine le ou les groupes phtaloyle et si on le désire

f) on convertit un composé obtenu répondant à la formule générale (I) en un sel acceptable pour l'usage pharmaceutique.

14. Médicament contenant un composé selon l'une des revendications 1 à 7.

15. Agent sédatif et anxiolytique actif contenant un composé selon l'une des revendications 1 à 7.

16. Agent antidépressif actif contenant un composé selon l'une des revendications 2 à 6.

**Revendications pour l'Etat contractant: AT**

1. Procédé de préparation des 3H-2-benzazépines de formule générale:

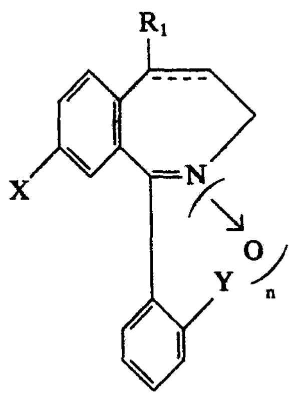

(I)

dans laquelle:
X représente l'hydrogène, le chlore ou le brome,
Y l'hydrogène, le fluor ou le chlore,
le trait interrompu une liaison facultative,
n est égal à 0 ou 1,
$R_1$ représente l'hydrogène, le brome, le chlore, l'iode ou un reste de formule:

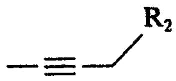

ou

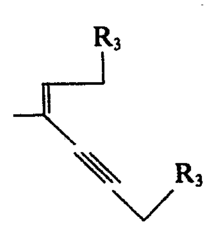

et

$R_2$ représente l'hydrogène, un groupe alkyle en $C_1-C_7$, hydroxy, amino, monoalkylamino en $C_1-C_7$ ou di-(alkyle en $C_1-C_7$)-amino et

$R_3$ représente un groupe hydroxy ou amino, étant spécifié que X et Y ne peuvent représenter tous deux l'hydrogène, et que le trait interrompu représente une liaison supplémentaire lorsque R ne représente pas l'hydrogène,

et de leurs sels acceptables pour l'usage pharmaceutique, caractérisé en ce que:

a) on cyclise un composé de formule générale:

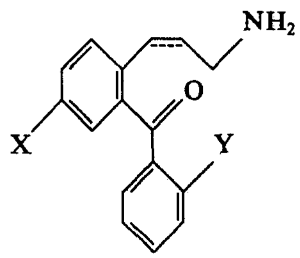

(II)

dans laquelle:
X et Y ont les significations indiquées ci-dessus et
le trait interrompu représente une liaison facultative, ou bien

b) on oxyde un composé de formule générale:

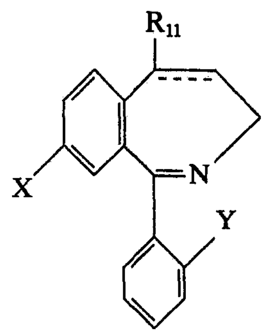

(Ia)

dans laquelle:
X et Y ont les significations indiquées ci-dessus,
$R_{11}$ repésente l'hydrogène, le brome, le chlore ou l'iode et
le trait interrompu une liaison facultative, étant spécifié que le trait interrompu représente une liaison supplémentaire lorsque $R_{11}$ ne représente pas l'hydrogène, ou bien

c) on soumet à deshydrohalogénation un composé de formule générale:

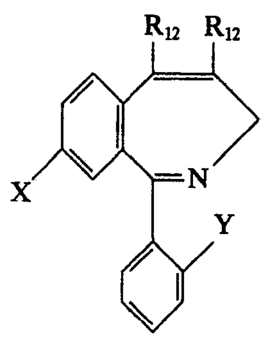

(III)

dans laquelle:
X et Y ont les significations indiquées ci-dessus et $R_{12}$ représente le chlore, le brome ou l'iode, ou bien

d) on fait réagir un composé de formule générale:

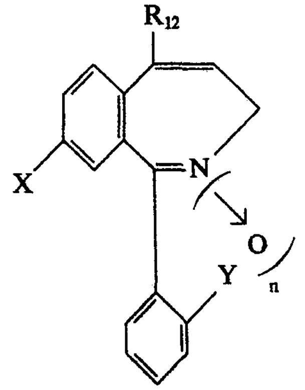

(Ib)

dans laquelle:
X, Y, n et $R_{12}$ ont les significations indiquées ci-dessus, en présence d'un sel de palladium, d'iodure de cuivre-I, d'une organophosphine et d'une amine secondaire ou tertiaire, avec un composé de formule générale:

$$HC \equiv C - CH_2 - R_{21} \qquad (IV)$$

dans laquelle:
$R_{21}$ représente l'hydrogène, un groupe alkyle en $C_1-C_7$, hydroxy, monoalkylamino en $C_1-C_7$ ou di-(alkyle en $C_1-C_7$)-amino, ou bien

e) dans un composé de formule générale:

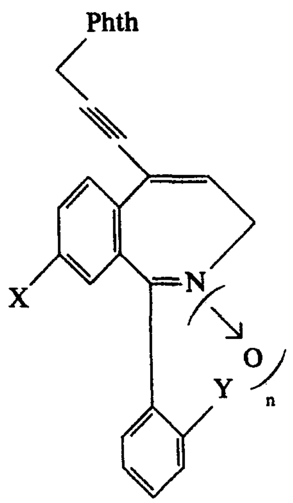

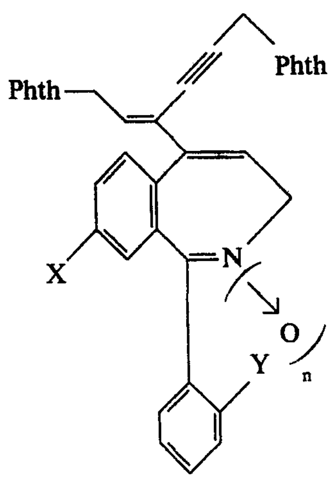

(V) ou (VI)

dans laquelle:
X, Y et n ont les significations indiquées ci-dessus et Phth représente un radical phtalimido, on élimine le ou les groupes phtaloyle, et si on le désire

f) on convertit un composé obtenu de formule générale (I) en un sel acceptable pour l'usage pharmaceutique.

2. Procédé selon la revendication 1, caractérisé en ce que l'on prépare un composé de formule générale:

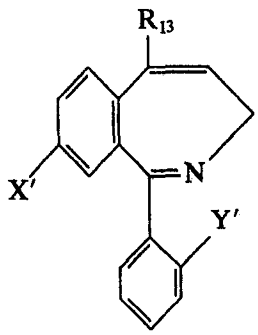

(Ie)

dans laquelle:
X' représente le chlore,
Y' l'hydrogène, le fluor ou le chlore,
$R_{13}$ un reste de formule:

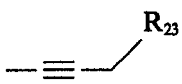

ou

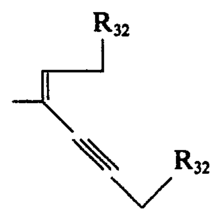

et
$R_{23}$ représente un groupe amino, monoalkylamino en $C_1-C_7$ ou di-(alkyle en $C_1-C_7$)-amino et
$R_{32}$ un groupe amino.

3. Procédé selon la revendication 2, caractérisé en ce que l'on prépare un composé de formule générale (Ie) définie dans la revendication 2, dans laquelle $R_{13}$ représente un reste de formule:

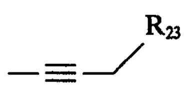

et $R_{23}$ a la signification indiquée dans la revendication 2.

4. Procédé selon la revendication 3, caractérisé en ce que l'on prépare la 8-chloro-5-(1-amino-2-propyne-3-yl)-1-(2-fluorophényl)-3H-2-benzazépine.

5. Procédé selon la revendication 3, caractérisé en ce que l'on prépare la 8-chloro-5-(1-amino-2-propyne-3-yl)-1-phényl-3H-2-benzazépine.

6. Procédé selon la revendication 3, caractérisé en ce que l'on prépare la 8-chloro-5-(1H-diméthylamino-2-propyne-3-yl)-1-(2-fluorophényl)-3H-2-benzazépine.

7. Procédé selon la revendication 1, caractérisé en ce que l'on prépare le 2-oxyde de 8-chloro-1-phényl-3H-2-benzazépine.